# EUROPEAN PATENT APPLICATION

(11) **EP 1 111 057 A1**
(43) Date of publication of application: **27.06.2001**
(21) Application number: 99940630.9
(22) Date of filing: 02.09.1999
(51) Int. Cl.: C12N 15/55, C12N 9/22, A61K 48/00

(54) **PRODRUG RIBOZYME**

(30) Priority: 03.09.1998 JP 24990098
(71) Applicant: Sumitomo Pharmaceuticals Company, Limited, Osaka 541-8510 (JP)
(72) Inventor: TOHDOH, Naoki;, Higashinada-ku, Kobe-shi, Hyogo 658-0056 (JP); YAMAMOTO, Hiroko, Higashiosaka-shi, Osaka 579-8058 (JP); SUDO, Yoshiaki, Nishinomiya-shi, Hyogo 662-0831 (JP)
(74) Representative: Cresswell, Thomas Anthony
(86) International application number: JP9904767
(87) International publication number: WO0014252

(57) **Abstract**

In accordance with the present invention, there is provided a gene encoding a prodrug ribozyme that is converted to a functional ribozyme in the cytoplasm after splicing, characterized in that said ribozyme contains, in the ribozyme sequence, an intervening sequence that is eliminated by splicing and said prodrug ribozyme has no RNA-cleaving activity.

## Description

### TECHNICAL FIELD

The present invention relates to a gene encoding a pharmaceutically useful prodrug ribozyme, an expression vector having said gene, and a pharmaceutical composition containing said gene as an active ingredient.

### BACKGROUND ART

In recent years, various therapeutic methods are used for the treatment of patients with various viral infections, including cytokine therapies (interferon therapy etc.), vaccine therapies, therapies using anti-viral agents based on inhibition of viral polymerases, proteases etc., and anti-viral agents employing nucleic acid analogs. Examples of severe viral diseases include AIDS, type B hepatitis, type C hepatitis, and the like. In the administration of various therapeutic agents to these patients with viral infections, appearances etc. of viral strains resistant to drugs are causing problems.

Since highly precise methods of detecting these viruses have recently been established, patients with post-transfusion infections are decreasing in number. In addition, as a result of prevention of mother-to-child infection by vaccination after diagnosis as in type B hepatitis, the ratio of virus carriers is on the decrease. However, the number of infected people in developing countries and in drug addicts is gradually increasing, representing grave social problems. Although the number of patients with post-transfusion infections is declining, it has been shown, patients infected with type C hepatitis virus (hereinafter referred to as HCV) amount to about 1% of the total population in the world.

HCV is a single-stranded RNA virus and thus is not integrated into the host's chromosome. Hepatitis due to HCV infection is a severe viral infection that develops into the chronic type at a high frequency, and then to liver cirrhosis to liver cancer. Furthermore, interferon-resistant HCV is widespread.

Hepatitis B virus (hereinafter referred to as HBV) is a double-stranded DNA virus that has been found to be integrated into the chromosome of the host organism, and accounts for the major causes of chronic hepatitis together with HCV, and thus its treatment is very important in order to prevent primary hepatoma. In addition, HBV is a severe infectious virus that causes symptoms of fulminant hepatitis at a certain frequency due to the mutation of the viral gene.

Human immunodeficiency virus (hereinafter referred to as HIV), a causative virus of AIDS, is a severe infectious virus that destroys the immunological ability of the patient after latent infection for several years, and causes opportunistic infection by cytomegalovirus etc., leading to patient's death. HIV infection due to transfusion has decreased but the virus is widespread throughout the world through intravenous drug injections and sexual intercourses. HIV is classified into the genus Lentivirus and is stably integrated into chromosomal DNA as lentiviruses are.

There are many other high-risk viruses present, including human papilloma virus and human herpes simplex virus, double-stranded DNA viruses suspected of their association with the onset of cervical cancer, influenza virus that is a single-stranded segmented RNA virus, and the like.

Virus is a foreign factor to the living body, and has a gene sequence that is not existed in the host's chromosome. This feature has been used in some methods to eliminate viral RNA. One method employs an antisense oligonucleotide, and another employs a ribozyme having an RNA-cleaving activity.

Ribozymes that catalyze the cleavage of RNA are roughly divided' into the large ribozymes and the small ribozymes (Eiko Ohtsuka, et al., Tanpakushitu Kakusan Koso (Protein, Nucleic Acid and Enzyme), 40: 1400-1407, 1995). Examples of the large ribozymes include Group I ribozymes represented by ribozymes involved in splicing of ribosomal RNA precursor of Tetrahymena, Group II ribozymes found in cellular organelles of eukaryotes, and RNaseP that catalyzes the processing of the 5'-end of tRNA. Examples of the small ribozymes include a hammerhead type ribozyme found in Avocado sunblotch viroid, a hairpin type ribozyme found in Tobacco ringspot virus, and the delta type ribozyme that takes the form of a pseudoknot structure found in a hepatitis virus, HDV. Among them, ribozymes applied in diseases are usually the hammerhead type and the hairpin type ribozymes.

Ribozyme is a low molecular weight RNA, and can be directly integrated into cells. various modifications thereof have been made since its stability is low in the blood and the cell. Nucleic acid-like substances such as ribozyme, though being low molecular weight, are very low in their frequency of being incorporated into the cell. Hence, in order to maximize the number of cells incorporated into the cell, very high blood levels must be maintained. Depending on the method of modification, there may be a risk that it becomes an antigen. Furthermore, in cases where the elimination of viral RNA expressed at high levels is desired, the cleavage of RNA by ribozyme administration cannot attain adequate therapeutic effects because the efficiency of being incorporated into the cell is extremely low.

On the other hand, when ribozyme molecules are expressed within the cell, it is possible to eliminate viral RNA by utilizing a promoter that expresses ribozyme at an amount sufficient to cleave viral RNA. However, when the amount of ribozyme expressed is too large, it is more likely that cell-derived RNA other than the viral RNA of interest is cleaved, thereby posing a threat of inflicting another damage to the host cell. Furthermore, if ribozyme molecules become an activated form after being transcribed in the nucleus, the ribozyme molecules may cleave cell-derived mRNA precursors different from the molecule of interest, irrespective of the presence of the target molecule of viral RNA in the cytoplasm. In this method, therefore, it is very likely that molecules other than the molecule of interest are cleaved than when ribozyme acts only in the cytoplasm.

Nevertheless, no means has been found to attain effective expression of ribozyme molecules that function only in the cytoplasm.

### DISCLOSURE OF THE INVENTION

It is an object of the present invention to provide a gene encoding a pharmaceutically useful prodrug ribozyme, an expression vector having said gene, a prodrug ribozyme, and a pharmaceutical composition comprising said gene as an active ingredient. More specifically, it is an.object of the present invention to provide a gene etc. encoding a prodrug ribozyme that is converted to a ribozyme being functional in the cytoplasm after splicing.

The gene expression from the chromosome of the cell or virus in the nucleus is usually initiated by transcription by RNA polymerase II. At this time, a segment from the transcription initiation point to downstream of the poly(A) addition signal is transcribed as a stretch of RNA to attach a cap structure to the 5'-end. Subsequently, an mRNA precursor (heterogeneous nuclear RNA: hnRNA) undergoes splicing so that intervening sequences are removed. The mature mRNA migrates from within the nucleus. through the nuclear pore to the cytoplasm, where translation is initiated. mRNA derived from the chromosome of'the cell containing an oncogene, or mRNA derived from DNA viruses undergoes the above processes and is present in the cytoplasm. Alternatively, viral RNA is converted to double-stranded DNA by reverse transcriptase, as is RNA synthesis of a retrovirus system inserted to chromosomal DNA. On the other hand, the gene of an RNA virus that is replicated by RNA-dependent RNA polymerase is not present in the nucleus but is directly translated in.the cytoplasm.

When ribozyme molecules are expressed in the nucleus and, for example, virus-derived RNA is targeted, it is very likely that cell-derived RNA is erroneously cleaved, though at a low frequency. Especially, since precursor RNA present in the nucleus contains many intervening sequences, it is believed, ribozyme molecules will cleave precursor RNA at a higher frequency than will cleave mature mRNA. Also, considering the effects on RNA viruses present only in the cytoplasm, ribozymes that become activated only in the cytoplasm are useful.

Thus, as a means to resolve the above problems, the present inventors have noted the fact that by inserting an intervening sequence (intron) in a ribozyme molecule, the intervening sequence comes to undergo splicing out during migration from the nucleus to the cytoplasm within the cell, and to function as a mature ribozyme only in the cytoplasm, and have found that by inserting an intervening sequence'into a ribozyme molecule, then ligating it to a suitable promoter, and then introducing the gene into a mammalian cell for expression therein, viral RNA molecules can be stably cleaved, and, therefore, have completed the present invention.

Thus, the gist of the present invention is as described in the following (1) to (22):
(1) a gene encoding a prodrug ribozyme having one or a plurality of the following features:
   (a) containing, in the ribozyme sequence, an intervening sequence that is eliminated by splicing, and
   (b) having no RNA-cleaving activity;
(2) the gene of the above (1) that is a small ribozyme selected from a hammerhead ribozyme, a hairpin ribozyme or a delta ribozyme, and a large ribozyme selected from Group I ribozyme, Group II ribozyme and RNaseP, or a derivative thereof;
(3) the gene of the above (2) in which the ribozyme is a small ribozyme selected from a hammerhead ribozyme, a hairpin ribozyme, and a delta ribozyme, or a derivative thereof;
(4) the gene of the above (2) in which the intervening sequence is present at the loop site or the stem site of the ribozyme sequence;
(5) the gene of the above (1) in which the intervening sequence is an intervening sequence derived from a virus or an intervening sequence derived from an eukaryote;
(6) the gene of the above (5) in which the intervening sequence is an intervening sequence derived from a virus selected from papovavirus, adenovirus, herpesvirus, papilloma virus, retrovirus, or lentivirus;
(7) the gene of the above (5) in which the intervening sequence is an intervening sequence derived from SV40, an intervening sequence derived from the rabbit β-globin gene, or an intervening sequence derived from the mouse albumin gene; and
(8) the gene of the above (1) in which the intervening sequence contained in the ribozyme sequence is flanked by a spacer sequence on both ends, wherein said spacer sequence comprises an exon partial sequence flanking the 5'-end and the 3'-end of said intervening sequence in a eukaryote or a virus from which said intervening sequence is derived;
(9) the gene of the above (1) in which a proximate region on both ends of the intervening sequence contained in the ribozyme sequence has the following sequence:
   (a) 5'-end terminal sequence: 5'-(A/C)AGGU(A/G)AGU
   (b) 3'-end terminal sequence:
      PyPyPyPyPyPyPyPyPyPyPyN(C/U)AG(G/A)-3' (Py represents U or C, and N represents any residue);
(10) the gene of the above (1) in which the ribozyme is a ribozyme having an activity of cleaving virus-derived RNA;
(11) the gene of the above (10) in which the ribozyme is a ribozyme having an activity of cleaving HCV RNA;
(12) the gene of the above (11) in which the ribozyme is a ribozyme that recognizes HCV RNA containing any of the sequences as set forth in SEQ ID NO: 42-50 or a sequence in which several bases thereof have been replaced;
(13) the gene. of the above (1) encoding a multiple prodrug ribozymes;
(14) the gene of the above (13) in which the intervening sequence contained in each prodrug ribozyme is different from one another;
(15) an expression vector having the gene of any of the above (1) to (14);
(16) the expression vector of the above (15) having a tissue specific promoter;
(17) the expression vector of the above (15) which contains a sequence having an activity of activating the extranuclear transport of mRNA or an activity of stabilizing mature mRNA;
(18) one or multiple prodrug ribozymes having the following features:
   (a) containing, in the ribozyme sequence, an intervening sequence that is eliminated by splicing, and
   (b) having no RNA-cleaving activity;
(19) a pharmaceutical composition comprising the gene of the above (1) as an active ingredient;
(20) a therapeutic agent for treatment of viral diseases comprising the gene of the above (10) as an active ingredient;
(21) a therapeutic agent for treatment of HCV diseases comprising the gene of the above (11) as an active ingredient; and
(22) a method of producing, in the cytoplasm, a mature ribozyme having an RNA-cleaving activity, said method comprising introducing the gene of the above (1) into an eukaryotic cell.

### BRIEF DESCRIPTION OF THE"DRAWINGS

Figure 1 shows the concept of a prodrug ribozyme containing an intervening sequence by way of a hammerhead ribozyme. Mature ribozyme molecules, when expressed in the nucleus, cleave cell-derived precursor RNA, whereas prodrug ribozyme molecules cannot. Prodrug ribozyme molecules, after undergoing splicing, become mature ribozyme molecules in the cytoplasm and then cleave target RNA molecules.

Figure 2 is a conceptual drawing of a method for constructing expression vectors containing prodrug ribozyme genes that contain intervening sequences. By way of example, a procedure of introducing an intervening sequence into a NotI cleavage site inserted into the ribozyme sequence is illustrated.

Figure 3 is a conceptual drawing of a method for detecting mature ribozymes and prodrug ribozymes. Prodrug ribozyme molecules from which intervening sequences have not been eliminated can be differentiated from mature ribozyme molecules by the RT-PCR method.

Figure 4 shows base sequences of 17 ribozyme molecules. Each of the underlined parts is a region homologous to the + strand RNA target sequence of NS5B.

Figure 5 is a drawing that shows building components of an expression plasmid containing HCV NS5B used for an in vitro transcription reaction.

Figure 6 is a photograph of electrophoresis that shows fragments of the reaction products between a ribozyme having a cleaving activity and its substrate HCV NS5B + strand RNA. Some results of the reaction of ribozymes having no cleaving activity are also included. Hereinafter, HCV NS5B + strand RNA was used as a substrate. Lane 2: Rz-f; Lane 3: Rz-g; Lane 4: Rz-h; Lane 5: Rz-1; Lane 6: Rz-m; Lane 7: Rz-n; Lane 8, 19: no ribozymes; Lane 10, 21: Rz-a; Lane 11, 23: Rz-b; Lane 13: Rz-i; Lane 14: Rz-j; Lane 15: Rz-k; Lane 16: Rz-o; Lane 17: Rz-p; Lane 18: Rz-q.

Hereinafter, HCV NS5B - strand RNA was used as a substrate. Lane 9, 20: no ribozymes; Lane 22: Rz-a; Lane 1, 12: an RNA size marker.

Figure 7 shows base sequences of six mature ribozyme molecules after intervening sequences were eliminated by splicing. Each of the underlined parts is a region homologous to the target sequence of HCV NS5B + strand RNA.

Figure 8 is a schematic diagram showing a mature ribozyme in which a restriction enzyme SalI recognition sequence or a restriction enzyme MaeIII recognition sequence has been inserted.

Figure 9 is a schematic diagram showing a mature ribozyme in which a restriction enzyme EcoRI recognition sequence has been inserted.

Figure 10 is a schematic diagram showing a mature ribozyme that contains no restriction enzyme cleavage sites.

Figure 11 are base sequences of six ribozyme molecules. Each of the underlined parts is a region homologous to the target sequence of HCV NS5B - strand RNA.

Figure 12 is a photograph of electrophoresis that shows fragments of the reaction products between a ribozyme and its substrate HCV NS5B - strand RNA. HCV NS5B - strand RNA was used as a substrate in Lanes 2-8. Lane 1: an RNA size marker, Lane 2: no ribozymes, Lane 3: cRz-a, Lane 4: cRz-b, Lane 5: cRz-c, Lane 6: cRz-d, Lane 7: cRz-e, and Lane 8: cRz-f.

Figure 13 is a photograph of electrophoresis that shows fragments of the reaction products-between a ribozymeRNA synthesized by in vitro transcription and its substrate HCV NS5B + strand. HCV NS5B + strand RNA was used as a substrate in Lanes 2, 3, 5, and 7. Lanes 4 and 6 show a ribozyme alone synthesized by in vitro transcription. Lane 1: an RNA size marker, Lane 2: no ribozymes, Lane 3: Rz-a (synthetic RNA product: positive control), Lane 4: Rz-a (no ribozymes), lane 5: Rz-a (ribozyme + substrate), Lane 6: Rz-a (Not-SV) (ribozyme alone), Lane 7: Rz-a (Not-SV) (ribozyme + substrate).

Figure 14 is a photograph of electrophoresis that shows fragments of the reaction products between a ribozyme and its substrate HCV NS5B + strand RNA. HCV NS5B + strand RNA was used as a substrate in Lanes 2-6. Lane 1: an RNA size marker, Lane 2: no ribozymes, Lane 3: Rz-a + Rz-b + Rz-n, Lane 4: Rz-a, Lane 5: Rz-b, Lane 6: Rz-n.

Figure 15 is a photograph of electrophoresis that shows fragments of the reaction products between a ribozyme and its substrate HCV NS5B + strand RNA. HCV NS5B + strand RNA was used as a substrate in Lanes 2-5. Lane 1: an RNA size marker, Lane 2: no ribozymes, Lane 3: Rz-a, Lane 4: Rz-a(-)S3, Lane 5: Rz-a(-)S4.

Figure 16 is a photograph of electrophoresis that shows fragments of the reaction products between a restriction enzyme recognition sequence-inserted ribozyme and its substrate HCV NS5B + strand RNA. HCV NS5B + strand RNA was used as a substrate in Lanes 2-4. Lanes 1, 5: an RNA size marker, Lane 2: no ribozymes, Lane 3: Rz-a, Lane 4: Rz-a(-)S6.

Figure 17 is a photograph of electrophoresis that shows fragments of the reaction products between a ribozyme synthesized by in vitro transcription and its substrate HCV NS5B + strand RNA. The two RNA fragments that were cleaved are indicated by arrows. HCV NS5B + strand RNA was used as a substrate in Lanes 2 and 4. Lanes 1 and 3 show the ribozyme alone that was synthesized by in vitro transcription. Lane 1: Rz-a(-)S (ribozyme alone), Lane 2: Rz-a(-)S (ribozyme + substrate), Lane 3: Rz-aS (ribozyme alone), Lane 4: Rz-aS (ribozyme + substrate), Lane 5: an RNA size marker.

Figure 18 shows a procedure of PCR for detecting NS5B RNA cleaved by ribozyme.

After mixing NS5B + strand RNA and ribozyme and performing a cleavage reaction, NS5B + strand RNA-expressing cell RNA is mixed, and a reverse transcription reaction is performed using an NSA-1 primer. After RNA is lysed with RNase and purified, an addition reaction of dC is performed. PCR is performed using a dC primer and a 2329R primer, and then a 1.1 kb DNA fragment derived from RNA that was not lysed with a ribozyme and a 0.5 kb DNA fragment that was lysed are subjected to electrophoretic separation.

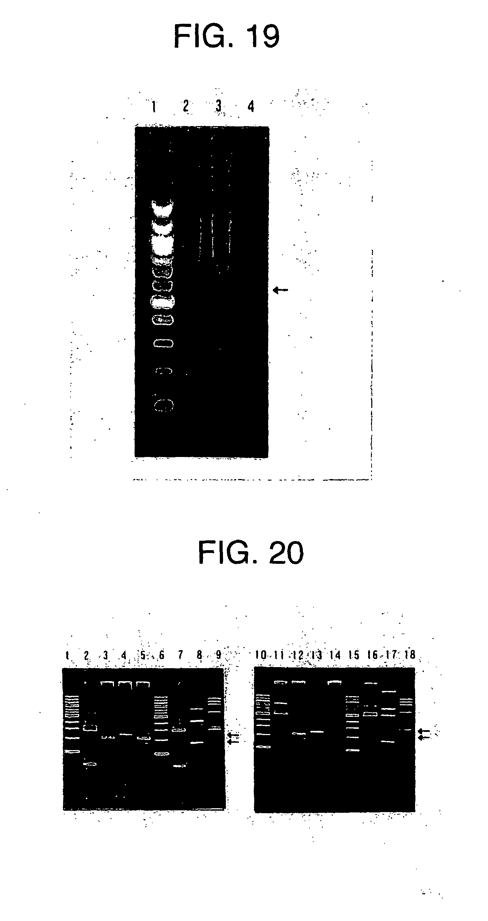

Figure 19 is a photograph of electrophoresis of products of PCR for detecting ribozyme cleavage fragments in the cell. Lane 1: an RNA size marker, Lane 2: RNA of NS5B + strand RNA-expressing cell + ribozyme-cleaved NS5B + strand RNA, Lane 3: RNA of NS5B + strand RNA-expressing cell + NS5B + strand RNA, Lane 4: no templates. In Lane 2, a fragment probably derived from ribozyme-cleaved RNA is confirmed at a position indicated by an arrow.

Figure 20 is a photograph of electrophoresis of products of PCR that investigated the splicing of intervening sequence of a ribozyme in the cell using various ribozyme-expressing plasmids. Lanes 2, 4, 7, 11,13, and 16 employ a prodrug ribozyme-specific primer, and Lanes 3, 5, 8, 12, 14, and 17 employ a mature ribozyme-specific primer. RNA derived from the cell in which the following plasmid was introduced was used, respectively. Lanes 2, 3: Rz-a(-)S3, Lanes 4, 5: Rz-aS3, Lanes 7, 8, 16, 17: Vector(pc△N), Lanes 11, 12: Rz-a(-)S4, Lanes 13, 14: Rz-aS4. Lanes 1, 6, 9, 10, 15, and 18 are an RNA size marker. In Lanes 4, 13, a fragment probably derived from prodrug ribozyme RNA is confirmed (upper arrow in the figure). In Lanes 3, 5, 12, and 14, a fragment probably derived from mature ribozyme RNA is confirmed (lower arrow in the figure).

### BEST MODE FOR CARRYING OUT THE INVENTION

The present invention will now be explained in further details hereinbelow.

The first embodiment of the present invention is a gene encoding one or multiple prodrug ribozymes having the following features:
(a) containing, in the ribozyme sequence, an intervening sequence that is eliminated by splicing, and
(b) having no RNA-cleaving activity;

As used herein, "ribozyme (RNA enzyme)" means RNA having an RNA-cleaving activity, and encompasses ribozymes naturally occurring in animals and plants, viruses, etc. and ribozyme derivatives artificially designed and produced with a reference to naturally occurring ribozymes. As naturally occurring ribozymes, there can be specifically mentioned the small ribozymes that include hammerhead ribozymes, hairpin ribozymes or delta ribozymes, and the large ribozymes that include Group I ribozymes, Group II ribozymes, or RNaseP (Eiko Ohtsuka, et al., Tanpakushitu Kakusan Koso (Protein, Nucleic Acid and Enzyme), 40: 1400-1407, 1995). Considering the simplicity of handling and ease of designing ribozymes, the small ribozymes that .include hammerhead ribozymes, hairpin ribozymes or delta ribozymes or the derivatives thereof are preferred, and the small ribozymes that include hammerhead ribozymes or hairpin ribozymes or the derivatives thereof are more preferred.

"Prodrug ribozyme" as used herein is RNA containing, in the ribozyme sequence, an intervening sequence that is eliminated by splicing and having no RNA-cleaving activity, and is also called ribozyme precursor. This ribozyme precursor is lacking an RNA-cleaving activity due to the insertion of an intervening sequence into the ribozyme sequence, and is converted to one having an RNA-cleaving activity by undergoing splicing in the cell leading to the elimination of the intervening sequence. In cases where this converted ribozyme is used distinguishably from prodrug ribozyme, this is called mature ribozyme. The mature ribozyme migrates from the nucleus to the cytoplasm, where it shows an RNA-cleaving activity.

Specifically, prodrug ribozymes in the following 1) and 2) are illustrated:
1) molecules in which an intervening sequence has been inserted into small ribozymes including the hammerhead type, the hairpin type and delta type or the derivatives thereof (small ribozyme precursors), and
2) molecules in which an intervening sequence has been inserted into large ribozymes including Group I type, Group II type and RNase P type or the derivatives thereof (large ribozyme precursors).

Preferably, there can be mentioned molecules in which an intervening sequence has been inserted into small ribozymes including the hammerhead type and the hairpin type or the derivatives thereof.

"Intervening sequences" as used herein may be any base sequences that are eliminated by splicing, and include those intervening sequences derived from viruses and eukaryotes. Furthermore, intervening sequences derived from plants can also be used. When a gene encoding the prodrug ribozyme of the present invention is administered for therapeutic purposes, the intervening sequence used is preferably derived from a species other than the patient, the animal or the plant to be treated, in order to minimize homologous recombination with chromosomal DNA of the patient, the animal or the plant to be treated. When humans are treated, an intervening sequence used is preferably derived from virus, or a mammal other than primates for example rabbits and rodents.

When virus-derived intervening sequences are used, virus of any source that has. the intervening sequence may be used. In order to secure splicing, it is preferred to select those intervening sequences for which no alternative splicing takes place. As the intervening sequences derived from viruses, there can be mentioned those intervening sequences of papovavirus, adenovirus, herpesvirus, papilloma virus, retrovirus, lentivirus and the like. As the intervening sequences derived from papovavirus, there can be mentioned intervening sequences containing proximate regions that are contained in early or late mRNA derived from SV40 virus. As the intervening sequences derived from lentivirus, there can be mentioned intervening sequences derived from HIV.

When intervening sequences of the gene contained in chromosomes of eukaryotes are used, the eukaryotes used are not limited. For example, there can be used intervening sequences derived from the rabbit β-globin gene or derived from the mouse albumin gene. When the base sequence of an intervening sequence is long, genes of the chromosome may be destroyed due to homologous recombination between the DNA introduced into the cell and the chromosomal DNA. Therefore, when it is intended to treat humans, especially when the intervening sequence is contained in a gene that is extremely important to the living body, it is preferred not to use a human-derived intervening sequence in order to reduce the frequency of homologous recombination by the intervening sequence.

Sites of ribozymes into which intervening sequences are inserted are those sites in which the inserted intervening sequences will destroy the conformation of ribozymes, with a result that an RNA-cleaving activity is lost. For those ribozymes that restore the cleaving activity after intervening sequences were excised by splicing, the intervening sequences may be inserted into any sites in the ribozyme molecules.

In order to obtain more pronounced effects of eliminating RNA-cleaving activity, intervening sequences may be inserted into those sites that are important for the RNA-cleaving activity of the ribozyme.

On the other hand, as described hereinbelow, it is preferred that restriction enzyme recognition sequences for facilitating the insertion of intervening sequences or spacer sequences for securing splicing do not affect the cleaving activity of mature ribozymes. For example, in the case of hammerhead type small ribozymes, intervening sequences are inserted into regions of the stem/loop structure that probably is not responsible for the cleaving activity (Eiko Ohtsuka, et al., Tanpakushitu Kakusan Koso (Protein, Nucleic Acid and Enzyme), 40: 1400-1407, 1995).

In accordance with the present invention, suitable restriction enzyme recognition sequences may be included in ribozymes in order to facilitate the insertion of intervening sequences. Many of the restriction enzyme recognition sequences have palindrome structures. Thus, restriction enzyme recognition sequences make possible the addition of a new stem structure to the ribozymes or replacement of the stem structure originally equipped in the ribozymes. In this case, restriction enzyme recognition sequences are preferably those that are not present in'the intervening sequences to be inserted.

In accordance with the present invention, spacer sequences may be retained at the sites of ribozymes into which intervening sequences are inserted in order to secure the splicing and the following elimination of the intervening sequences. In eukaryotes or viruses from which intervening sequences are derived, spacer sequences are exon partial sequences that flank said intervening sequences at the 5'-end and the 3'-end, and each of them preferably has two to a few dozen bases, more preferably 2-10 bases. In prodrug ribozymes, spacer sequences are located in both regions that flank the intervening sequences. The both regions that flank the intervening sequences refer to those regions that are not contained in the intervening sequences but that are adjacent to the 5'-end and the 3-end of the intervening sequences.

In order to initiate splicing, prodrug ribozymes may be designed so as to contain a proximate sequence (5'- (A/C)AG[GU(A/G)AGU ... PyPyPyPyPyPyPyPyPyPyPyN(C/U)AG] (G/A)-3': inside of [] is an intervening sequence, Py represents U or C, and N represents any base) (Alberts B., et al., Kyoikusha, Saibono Bunsiseibutugaku (Molecular Biology of the Gene) Second Edition, p534, 1990) of an intervening sequence found in many genes. In prodrug ribozymes, this sequence is located in the proximate region at both ends of the intervening sequence. The proximate region at both ends of the intervening sequence means a region that is near the 5'-end terminal or the 3'-end terminal of the intervening sequence and that contains part of the both ends of the intervening sequence. Having this sequence is thought to be associated with the occurrence of splicing by snRNA (small nuclear RNA) as represented by U1 RNA in the nucleus (Tokio Tani, et al., Tanpakushitu Kakusan Koso (Protein, Nucleic Acid and Enzyme), 40: 1408-1420, 1995). Furthermore, those genes that do not conform to the general sequence for the above splicing can also be used for the present invention.

As the target RNA for which ribozymes exhibit a cleaving activity, there can be mentioned virus-derived RNA, oncogene RNA that underwent mutation, deletion, or addition, etc. Examples of viruses include HCV, HBV, HIV, influenza virus, and the like.

Now, the method of preparing a gene encoding the prodrug ribozyme of the present invention is described. First, the method of preparing ribozyme for inserting an intervening sequence is described.

### (1) Determination of the target sequence for cleaving mRNA

As the target sequence for cleaving mRNA, any region may be selected. However, since ribozyme molecules are expected to have a high cleaving activity to the base sequence- in the loop portion of the secondary structure of mRNA (Sakamoto N. et al., J. Clin. Invest. 98: 2720-2728, 1996), computer analysis is conducted to assemble the secondary structure of mRNA and then any target sequence of the loop portion is preferentially selected.

### (2) Designing of a ribozyme and construction of a ribozyme molecule

Designing of a ribozyme may be carried out by designing a base sequence of any length that anneals to the substrate mRNA and then by inserting a cleaving activity site of the ribozyme into said base sequence. The sequence required for cleaving activity of ribozymes may be determined based on known sequences (for example, Kijima H, et al., Pharmac. Ther., 68: 247-267, 1995, or Eiko Ohtsuka, et al., Tanpakushitu Kakusan Koso (Protein, Nucleic Acid and Enzyme), 40: 1400-1407, 1995). Ribozyme molecules for investigating an in vitro cleaving activity may be prepared by RNA synthesis. Alternatively, a DNA fragment encoding-the ribozyme may be inserted into an appropriate vector, which is in vitro transcribed to prepare a ribozyme molecule. When the number of base sequences is small, it is easier to prepare ribozyme molecules by RNA synthesis.

### (3) Evaluation of the in vitro cleaving activity of the prepared ribozyme

By mixing the substrate RNA and a ribozyme, the in vitro cleaving activity may be evaluated. As the substrate RNA, total RNA or poly (A)RNA derived from the cell that expresses the target mRNA may be used. The preparation of RNA from the cell may be carried out according to a standard method. In this case, a ribozyme is mixed in vitro. After a cleaving reaction, the cleavage of the target mRNA can be confirmed by Northern hybridization using a DNA fragment encoding the target mRNA as a prude, PCR or the like. RNA used as the substrate may be prepared by an in vitro transcription reaction using an appropriate plasmid vector that contain cDNA encoding the target mRNA. The in vitro transcription reaction could be easily carried out using a commercial kit, and the reaction can be carried out by T7 RNA polymerase, T3 RNA polymerase or SP6 RNA polymerase, which is suitable for the inserted vector. In this case also, Northern hybridization may be used for analysis as when total RNA or poly(A)RNA is used, but more simply, the 5'-end of RNA may be labeled with RI or fluorescence and after electrophoresis the cleaving activity may be directly analyzed. In this case, it is common to label with ³²P or biotin. When RNA is labeled, a base labeled (for example, with [α-³²P]UTP) during the in vitro transcription may be incorporated into RNA. A simpler method is one that incorporates a base labeled during in vitro transcription into RNA since it enables to obtain a highly purified and homogenous substrate RNA. Cleaving may be conducted using a reaction system described in a literature (Kanazawa Y. et al, Biochem. Biophys. Res. Commun. 225: 570-576, 1996).

### (4) Investigation of specificity of cleaving activity to the target mRNA

In order to investigate the specificity of cleaving activity of ribozymes, cell-derived total RNA or poly(A)RNA is added at the same time as the cleaving reaction of labeled RNA synthesized by in vitro transcription, and then the absence of changes in the cleaving activity to the labeled target mRNA is confirmed. At this time, changing, stepwise, the amount of RNA added to the reaction system is also important in evaluating the cleaving activity. Alternatively, an oligonucleotide probe to the portion in the ribozyme that anneals to the target mRNA may be prepared, which is used to confirm that there is no RNA, in the cell, having a region homologous to cell-derived total RNA or poly(A)RNA by Northern hybridization, PCR or the like. Oligonucleotides can be obtained by DNA synthesis, and generally 5'-end-labeled ones are used. By these experiments, it is confirmed that the action site of the designed ribozyme is specific for the target mRNA.

### (5) Introduction of a ribozyme molecule having a cleaving activity into an expression vector and DNA transfection into cultured cells that express the target mRNA

In order to express a ribozyme molecule having an in vitro activity of cleaving the target mRNA, a double-stranded DNA compatible with the ribozyme molecule is synthesized. ' Double-stranded DNA may also be obtained by preparing a primer in which a sequence for a restriction enzyme has been ligated to the 5'-end and amplifying it by PCR. In this case, a ribozyme RNA molecule may be used as a template. Alternatively, it is also possible to design primers so that they anneal to each other at the 3'-end, and to amplify between the primers alone.

In any method, it is desirable to make the both ends of DNA corresponding with the restriction enzyme sites to be inserted into a vector to facilitate the integration into the plasmid vector. When amplified using a PCR reaction, it is cleaved with a suitable restriction enzyme and then subjected to acrylamide gel electrophoresis, gel filtration, ultrafiltration or the like to purify the DNA fragment of interest. After treatment with a restriction enzyme, the DNA fragment of interest-may be ligated using T4 DNA ligase to an expression plasmid vector such as pcDNA3.1(+) that has been dephosphorylated using alkaline phosphatase. Thereafter, the expression plasmid vector obtained may be introduced into competent cells of E. coli such as JM109 to obtain a transformed E. coli that is ampicillin-resistant. Then, plasmid DNA is recovered from a plurality of clones obtained and the correct insertion of the DNA of interest into the plasmid vector by restriction enzyme treatment is confirmed by acrylamide electrophoresis etc. Alternatively, the detection of inserted 'fragments may be carried out using the PCR method.

Expression vectors containing ribozyme genes may be introduced into cultured cells using a simple method such as the liposome method, the lipofection method, the calcium phosphate co-precipitation method, and the electroporation method. In this case, the cultured cells may be those that originally express the target mRNA, or those that express the target mRNA as a result of transformation using a vector that expresses the target RNA.

When a viral base sequence etc. is targeted, it is preferred to obtain in advance a stably transformed cell line by preparing a recombinant expression vector and then introducing it into cultured cells, since the common cultured cells do not express the gene of interest. Viral DNA fragments, in the case of RNA viruses, may be amplified by RT-PCR, or in the case of DNA viruses, amplified by PCR. But, when it is integrated into chromosomal DNA by reverse transcriptase activity as for viruses of the retrovirus system, it is possible to amplify from the chromosomal DNA of the infected cells. When DNA fragments are to be obtained by the PCR reaction, it is desirable to add a restriction enzyme recognition sequence suitable for insertion to both ends of DNA. Primers are synthesized so as to contain a restriction enzyme recognition sequence at the 5'-end side of the primer used for PCR, in addition, it is possible to ligate a linker or an adapter to the both ends of DNA using T4 DNA ligase after amplifying DNA with a primer consistent with the viral base sequence. When virus particles are obtained in large quantities, the viral DNA may be directly cleaved with a restriction enzyme, and then the DNA fragment of interest may be obtained. The virus-derived DNA thus obtained may be inserted into an appropriate expression vector such as pBK-CMV to obtain a recombinant expression vector. Then it may be introduced into a suitable cultured cell using a simple method such as the liposome method, the lipofection method, the calcium phosphate co-precipitation method, and the electroporation method. Depending on the type of expression vector used, a drug (neomycin in the case of pBK-CMV) is added to the culture liquid, and the transformed cells that exhibit drug resistance are selected. Subsequently, from each clone obtained, DNA, RNA, or protein is collected, and then transformed cells suitable for the evaluation of cleaving activity in the cultured cell of ribozymes are selected using Southern hybridization, Northern hybridization, PCR, PT-PCR, or antibody against viral protein encoded by viral DNA introduced into an expression vector. The transformed cells to be selected are preferably those in which viral RNA and virus-derived protein are evidently detected using Northern hybridization or antibody.

### (6) Evaluation of cleaving activity by ribozyme in cultured cells

The cleaving activity of ribozyme in cultured cells may be investigated by preparing total RNA or poly(A)RNA from cultured cells cultured for several days after the introduction of a ribozyme expression vector and then by Northern hybridization using a DNA probe suitable for detection of the target RNA, PCR or the like. As the DNA probe, there can be used one in which the 5'-end of DNA was labeled with T4 DNA kinase using [γ-³²P] ATP after treatment with E. coli alkaline phosphatase, or one labeled by the nick translation method using E. coli DNA polymerase I and [α-³²P]dNTP or by the random prime labeling method using random primers. It is most simple to prepare a DNA probe using the random prime labeling method.

When the amount of target RNA is small in cultured cells, the activity may be investigated by designing primers that may be specifically amplified using the target RNA as a template, and then by detecting DNA fragments derived from non-cleaved RNA using as a template total RNA or poly(A)RNA derived from cultured cells into which a ribozyme expression vector has been introduced.

Next, the method of preparing a gene encoding a prodrug ribozyme in which the intervening sequence of the present invention has been inserted is described.

### (1) Construction of a vector expressing a prodrug ribozyme gene into which an intervening sequence has been inserted

In an expression vector containing ribozyme for which the cleaving activity was observed in the cultured cell, an intervening sequence may be inserted into any site in the DNA encoding the ribozyme to obtain a vector that expresses a prodrug ribozyme gene.

The intervening sequence may be inserted into any site of the ribozyme molecule if the ribozyme molecule can restore the cleaving activity after the excision of the intervening sequence by splicing, but it is simple to insert it into a site that does not affect the cleaving activity of mature ribozyme. For example, in the case of a hammerhead type small ribozyme molecule, an intervening sequence may be inserted into a region in the stem/loop structure that is considered not to affect the cleaving activity (Eiko Ohtsuka, et al., Tanpakushitu Kakusan Koso (Protein, Nucleic Acid and Enzyme), 40: 1400-1407, 1995). In this case, the insertion of the intervening sequence is easier if there is a cleavage site by a restriction enzyme in advance to facilitate insertion of the intervening sequence into the stem/loop structure. Since many of the restriction enzyme recognition sequences have palindrome structures, restriction enzyme cleavage sequences may be included in the stem.

Generally, splicing from an mRNA precursor to a mature mRNA in the cell is catalyzed by snRNA represented by U1-5 in the nucleus. When a general mRNA intervening sequence such as this is used, one can use a general sequence in the boundary region of splicing (5'-(A/C)AG[GU(A/G)AGU ...... PyPyPyPyPyPyPyPyPyPyPyN(C/U)AG](G/A)-3': inside of [] represents an intervening sequence (intron), Py represents U or C, and N represents any base). First, as the primer at the 5'-end, a sequence is prepared that contains at least a general sequence (A/C)AGGT(A/G)AGT at the 5'-end boundary and that has added, to the 5'-end, a restriction enzyme sequence inserted into a DNA molecule encoding ribozyme. Furthermore, as the 3'-end primer, a sequence is prepared that contains at least a general sequence (T/C)CT(A/G)NPuPuPuPuPuPuPuPuPuPuPu (Pu represents A or G, and N represents any base) at the 3'-end boundary and that has added, to the 5'-end, a restriction enzyme sequence inserted into a DNA molecule encoding ribozyme. Then, by PCR using the both sequences as primers, a fragment containing an intervening sequence may be amplified with the chromosomal DNA as a template.

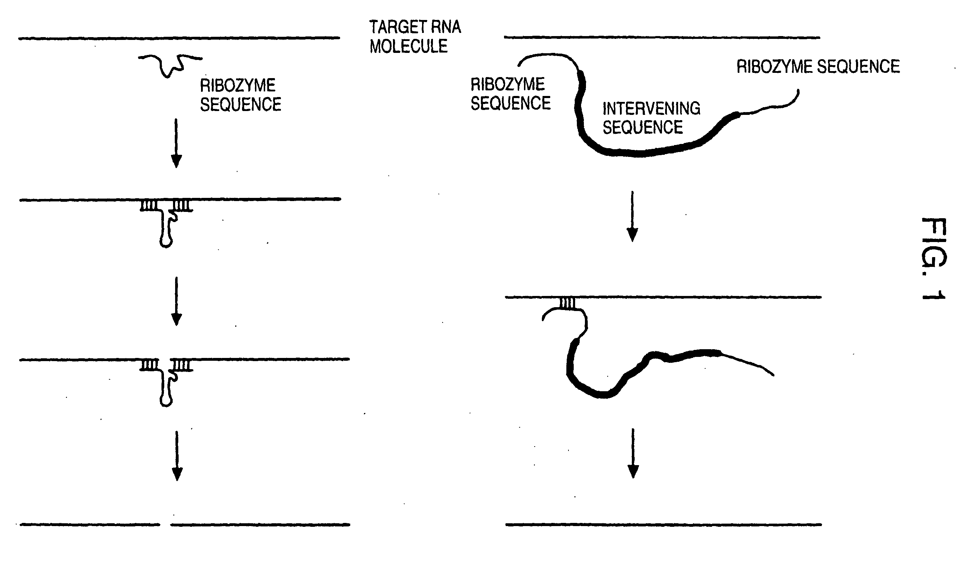

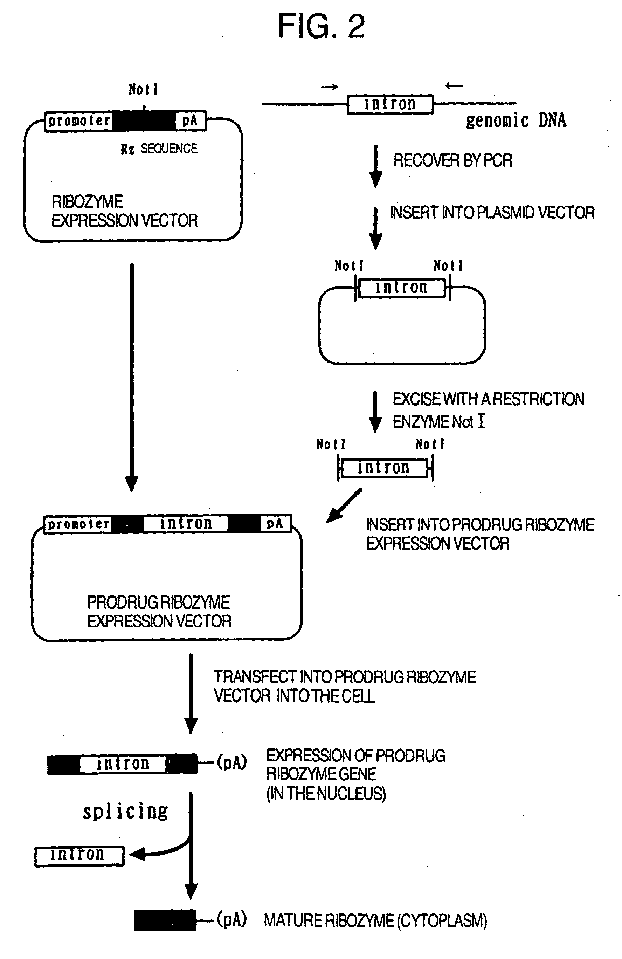

After cleaving the DNA fragment containing the amplified intervening sequence with a restriction enzyme, it is ligated to a restriction enzyme recognition site in the DNA encoding the ribozyme by T4 DNA ligase. When the intervening sequence is short, the insertion sequence may be prepared by DNA synthesis, which is then inserted directly into the restriction enzyme recognition site in the DNA encoding the ribozyme. The conceptual drawing of the prodrug ribozyme molecule containing an intervening sequence thus constructed is illustrated by way of a hammerhead type small ribozyme in Figure 1. A simple procedure for preparation is illustrated in Figure 2 for the case in which a NotI restriction enzyme recognition sequence is used.

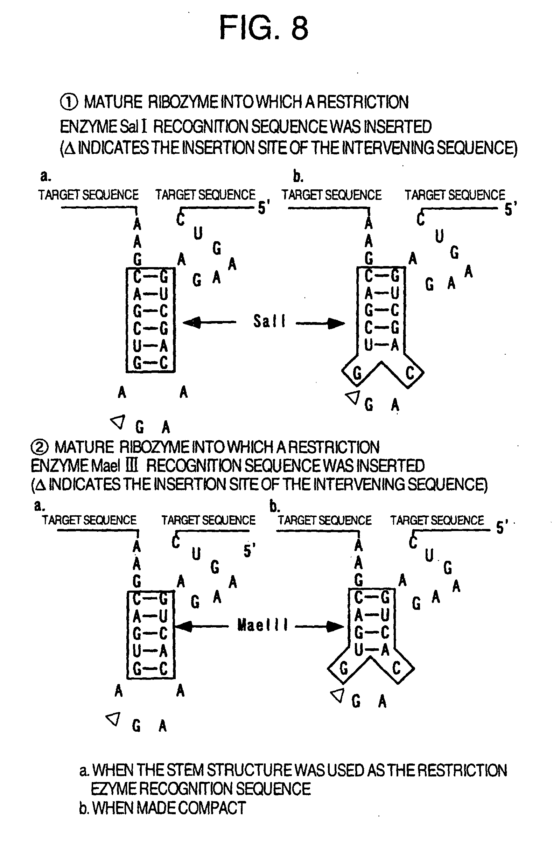

Furthermore, one example of a ribozyme molecule that has a restriction enzyme cleavage sequence inserted using the stem structure is shown in Figure 8. In this case, in order to make the ribozyme molecule compact, the junction region sequence for splicing, i.e. (A/C)AG∇(G/A) sequence may be overlapped with the restriction enzyme cleavage sequence. As used herein, ∇ means a site into which an intervening sequence may be inserted. Figure 8 illustrates the examples of a restriction enzyme SalI that recognizes six bases and a restriction enzyme MaeIII that recognizes five bases, and shows a mature ribozyme sequence from which an intervening sequence has been eliminated by splicing when all restriction enzyme sequences were inserted into the stem structure and when the mature ribozyme was made compact using the' boundary region of the intervening sequence. When the stem structure has all restriction enzyme sequences, a longer proximate sequence of the intervening sequence can be easily inserted into the loop region.

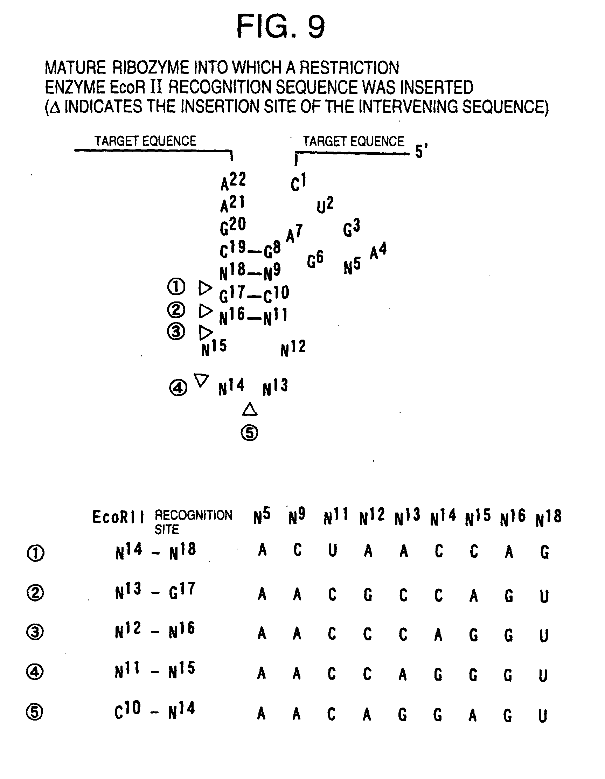

The position of a restriction enzyme cleavage sequence is not restricted to the stem structure but may be inserted to various regions. In this case also, a junction region sequence for splicing may be used to make a mature ribozyme molecule compact. Figure 9 illustrates an example in which a restriction enzyme EcoRII that recognizes five bases was used. As in a mature ribozyme shown in Figure 9, a site into which an intervening sequence is inserted may be set up not only in the loop region but in the stem structure. When an intervening sequence is inserted into the stem structure, it is thought to be difficult to retain the structure of the original ribozyme as compared to when the intervening sequence was inserted into the loop structure. Thus, intervening sequences may be inserted into various sites using restriction enzyme cleavage sequences.

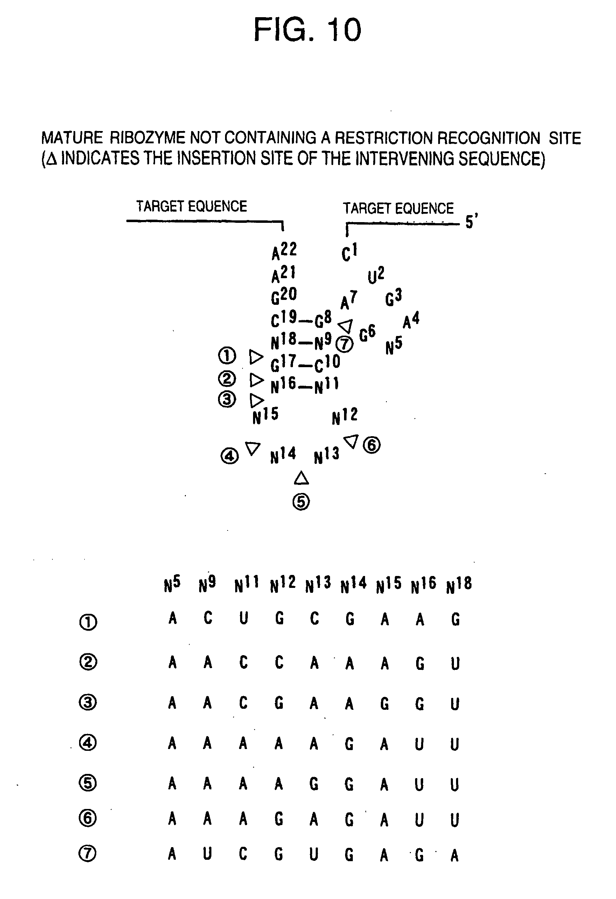

It is also possible to insert an intervening sequence without a restriction enzyme cleavage sequence. For example, as shown in Figure 10, an insertion site for an intervening sequence may be freely set up by inserting a suitable base into a site of base indicated by N. In this case, insertion may be effected into the loop region, the stem structure and a region essential for activity.

When a restriction enzyme recognition sequence is not used, a proximate sequence of the intervening sequence, a ribozyme sequence, and a restriction enzyme recognition sequence for insertion into a vector may be added to the 5'-end of the PCR primer sequence to amplify the inserted sequence, and after cleaving with a restriction enzyme, it can be inserted into a vector.

### (2) Introduction of a vector expressing a prodrug ribozyme gene into a cultured cell expressing the target mRNA

The-constructed vector expressing a prodrug ribozyme gene is introduced into a cultured cell expressing the target mRNA mentioned in the above (5) by DNA transfection. As the method of introduction, as in the above (5), a simple method such as the liposome method, the lipofection method, the calcium phosphate co-precipitation method, and the electroporation method may be used.

### (3) Confirmation that a mature ribozyme is being produced in cultured cells

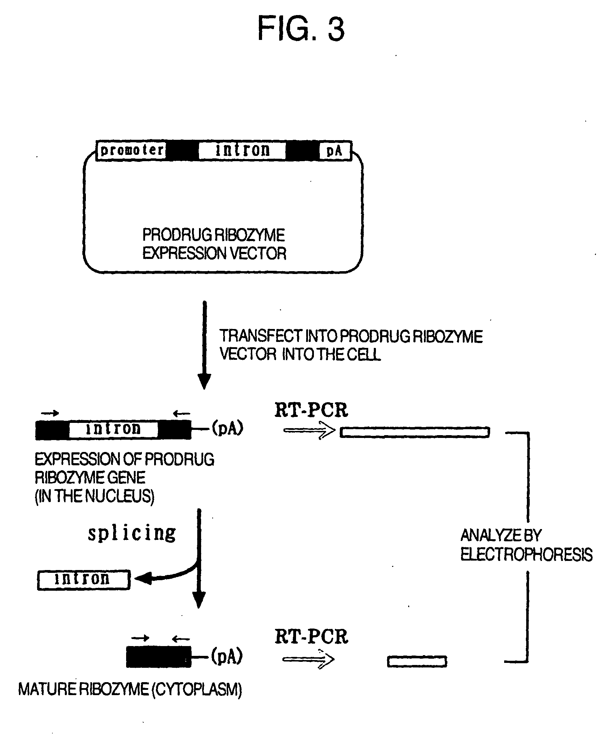

The fact that a mature ribozyme is produced by splicing in cultured cells can be confirmed by RT-PCR using primers that amplify the expressed mature ribozyme RNA. In this case, the primer used may be any primer that is within an expression unit, and any of a sequence in the ribozyme, a sequence existing from the transcription initiation site to the ribozyme, or an upstream sequence from the site where poly(A) is added may be used. After amplification, acrylamide gel electrophoresis etc. may be used for detection. By setting up suitable primers located in an intervening sequence region, and in between a ribozyme region and vector, RNA molecules in the cytoplasm that did not undergo splicing may be detected. In this case, after amplification by RT-PCR using appropriate combinations of a primer sequence which is located in ribozyme molecules, from the transcription initiation site to the ribozyme, or in the upstream sequence from the site where poly(A) is added, with a primer sequence in the intervening sequence, acrylamide gel electrophoresis etc. may be used for detection. Figure 3 shows a conceptual drawing of a method in which a primer sequence that detects a mature ribozyme or a prodrug ribozyme molecule is set up and of a method for confirming that mature ribozyme is being produced by splicing in cultured cells.

Furthermore, using total RNA or poly(A)RNA, it is possible to detect mRNA of ribozyme molecule, and molecule remaining intervening sequences by Northern hybridization using as a probe DNA encoding ribozyme or DNA encoding a prodrug ribozyme containing an intervening sequence, PCR or the like.

Prodrug ribozyme of the present invention is existing as"prodrug ribozyme mRNA in the nucleus and as mature ribozyme mRNA in the cytoplasm, and thereby the elimination of the nucleus is very important so as not to be contaminated with nuclear RNA when RNA is extracted from the cell. Generally RNA in the cytoplasm may be recovered by gently solubilizing the cell and then precipitating the nucleus by centrifugation. The method used may be published one (Nobuo Nomura, Jikkenn Igaku (Experimental Medicine), Supplement, Sin-idenshiKogaku Handbook (New Gene. Engineering Handbook), pp. 18-21, 1996) in which RNA in the cytoplasm may be prepared, or a commercial kit may be available.

### (4) Evaluation of cleaving activity by ribozyme in cultured cells

In order to investigate cleaving activity by ribozymes in cultured cells, total RNA or poly(A)RNA is prepared from the cells cultured for several days after introduction of a prodrug ribozyme expression vector, as in the above (6), and Northern hybridization using a DNA probe suitable for detection of the target RNA, PCR or the like is performed for investigation of the activity. Alternatively, by setting up primers that specifically amplify using as a template the target RNA in cultured cells, and using total RNA or poly(A)RNA as a template in the cultured cells into which a ribozyme expression vector has been transfacted, DNA fragments derived from RNA that was not cleaved are detected to investigate the activity.

The gene of the present invention may encode not only one but a plurality of prodrug ribozymes. In particular, the use of a gene encoding a plurality of prodrug ribozymes is effective for viruses having a high frequency of mutation, that is, a high frequency of appearances of resistant viruses. Specifically, it is effective for elimination of RNA derived from HIV or HCV.

The production of ribozymes using a plurality of prodrug ribozymes may be effected by ligating not only prodrug ribozyme molecules having one type of intervening sequence but also a plurality of prodrug ribozymes having different intervening sequences, and then by expressing suitable-genes in the cell. In this case, if intervening sequences contained in a prodrug ribozyme to be ligated are identical, they have a risk of undergoing splicing in different prodrug ribozyme molecules, and therefore, it is preferred that each of prodrug ribozymes have a different intervening sequence.

In a specific example, an SV40-derived sequence may be used as an intervening sequence for the first prodrug ribozyme molecule, a rabbit β-globin gene-derived sequence may be used as an intervening sequence for the second prodrug ribozyme molecule, and a mouse albumin gene-derived sequence may be used as an intervening sequence for the third prodrug ribozyme molecule. The combination or arrangement of the intervening sequences used may not be limited unless alternative splicing takes place.

More specifically, each DNA encoding a prodrug ribozyme containing an intervening sequence is excised from a recombinant plasmid vector, which is then sequentially inserted into a plasmid having a multi-cloning site such as pBluescriptII to prepare a recombinant vector containing contiguous prodrug ribozyme sequences. The DNA fragments containing the constructed entire contiguous prodrug ribozyme is excised and inserted into a suitable expression vector. Restriction enzyme recognition sequences for inserting an intervening sequence into each ribozyme sequence may be the same or different for each ribozyme, but restriction enzyme recognition sequences for inserting a prodrug ribozyme DNA molecule into the vector must be different from one another.

The gene encoding the prodrug ribozyme of the present invention can be transcribed not only by RNA polymerase III but also by RNA polymerase II because it has an intervening sequence and thereby has an increased number of bases of precursor RNA molecules to be transcribed. In order for the gene to be transcribed with RNA polymerase II, the number of bases in the intervening sequence is preferably about 500 bp or more, when the sequences other than the intervening sequence are short.

If ribozyme causes, even only slightly, side effects by the cleavage of RNA derived from the cells other than the target molecule, the expression locus should be restricted to a specific tissue or a disease site. However, there is no tissue specificity observed in the expression of ribozyme molecules. Ribozyme molecules are low molecular weight RNA, and are usually produced by transcription by RNA polymerase III, and thereby a tRNA promoter or the adenovirus VA1 promoter is used for the expression of ribozyme molecules. It is because these promoters have no tissue specificity. On the other hand, in the case of expression by RNA polymerase II, there is a specificity in the expression of ribozyme molecules and thus the tissue specificity of promoters can be utilized.

Furthermore, by being transcribed by RNA polymerase II, stability in the nucleus or migration into the cytoplasm can be secured. See the following for details.
(a) Prodrug ribozyme molecules that contain intervening sequences and that have poly(A) sequences added by the reaction of RNA polymerase II become stabilized by forming a hnRNP (heterogeneous nuclear ribonucleo-protein) complex in the nucleus (Patton JR, et al., Mol. Cell Biol., 5: 1220-1228, 1985), and form a complex with spliceosome around the nuclear pore. By such complex formation, prodrug ribozyme molecules do not undergo common RNase digestion and thus are stably retained in the nucleus.
(b) By the addition of poly(A) subsequently to the reaction of RNA polymerase II, exonucleases are inhibited in the direction of 3' to 5' thereby creating resistance to RNase (Ford LP, et al., Mol. Cell Biol., 17: 398-406, 1997), and the cap structure added to the 5'-end at the time of transcription by RNA polymerase II also contributes to the stabilization of RNA (Coutts M. et al., Biochim. Biophys. Acta., 1173: 49-56, 1993).
(c) The cap structure at the 5'-end (Mutsuto Ohno, Jikkenn Igaku (Experimental Medicine), 14: 38-44, 1996) and the poly(A) sequence (Huang Y. et al., Mol. Cell Biol., 16: 1534-1542, 1996) of mRNA facilitates migration from the nucleus to the cytoplasm.

In accordance with the present invention, intervening sequences are inserted into ribozyme molecules. Through this, not only the transcription by RNA polymerase II be effected but the following advantages may be obtained. The following advantages cannot be obtained when any conventional gene sequence is inserted into upstream or downstream of ribozyme molecule to lengthen the transcription products.
(a) The RNA cleaving activity in the nucleus can be substantially reduced. Thus, by steric hindrance by proteins such as hnRNP bound to the prodrug ribozyme molecule, the activity of cleaving cell-derived precursor RNA molecules are expected to be substantially reduced, and at the same time it becomes difficult to assume the ribozyme structure having an RNA-cleaving activity because the intervening sequence itself that was inserted into the ribozyme molecule takes a secondary structure and free energy drops by the insertion of intervening sequences having a large number of bases as compared to ribozyme molecules (Susan MF. et al., Proc. Natl. Acad. Sci. USA, 83: 9373-9377, 1986, Papanicolaou C. et al., Nucleic Acids Res., 12: 31-44, 1984). The effect becomes more pronounced by inserting an intervening sequence into sites important for the RNA-cleaving activity.
(b) By inserting an intervening sequence into a ribozyme, it becomes possible to extremely curtail the sequence up to poly(A) that is added to the 3'-end of the ribozyme gene region having an intervening sequence. Ribozyme molecules after being spliced become the activated form that has an addition of few extra base sequences. In order for the ribozyme molecule to retain a stable cleaving activity, it is very important that extra sequences may not be added, as much as possible, to the mature ribozyme molecules produced by splicing from the prodrug ribozyme.
(c) By inserting an intervening sequence into the ribozyme molecule of interest as in the present invention, the amount expressed of the gene can be increased (Brinster RL. et al, Proc. Natl. Acad. Sci. USA, 85: 836-840, 1988), and, at the same time, the length to the cap site 5' upstream of the ribozyme molecule and the length to the poly(A)-added signal 3' downstream can be freely selected. As a result, DNA fragment having a poly(A)-added signal of any gene may be used. Without being restricted to DNA fragments etc. containing a poly(A)-added signal derived from SV40 that contains an intervening sequence as do conventional expression vectors, suitable DNA fragments containing a poly(A)-added signal may optionally be selected. Specifically, DNA fragment that contain chicken β-actin poly(A) additional signal may be used. DNA fragments that contain a sequence responsible for the stabilization of mRNA and a poly(A)-added signal but that do not contain intervening sequences may also be readily used.

The second embodiment of the present invention is an expression vector having the gene that is the first embodiment of the present invention.

The expression vectors of the present invention may be roughly divided into two: the non-viral vectors and the viral vectors. As the non-viral vectors, there can be mentioned mammalian expression plasmid vectors. As the viral vectors, there can be mentioned viral vectors such as adenovirus and retrovirus. More specifically, DNA viruses such as detoxified retrovirus, adenovirus, adeno-associated virus, herpesvirus, vaccinia virus, poxvirus, poliovirus, Sindbis virus, Sendai virus, SV40, and human immunodeficiency virus (HIV), or RNA viruses.

By including a tissue-specific promoter and/or an enhancer into the expression vector of the present invention, ribozymes may be expressed in a tissue-specific manner. It is possible to use only one promoter or to combine a plurality of promoters and enhancers or silencers.

Tissue-specific promoters as used herein are promoters that exhibit a promoter activity only in specific tissues. For example, when it is desired to express a prodrug ribozyme molecule specifically in the liver, there can be mentioned the promoter of human albumin gene (Hayashi Y. et al., J. Biol. Chem., 267: 14580-14585, 1992), and as virus-specific ones, there can be mentioned, when HIV is targeted, promoters that are specifically expressed in helper T cells or macrophages. As the promoter that is specifically expressed in helper T cells, promoters such as CD4 may be used (Salmon P. et al., Proc. Natl. Acad. Sci. USA, 90: 7739-7743, 1993). Also, as the promoter that is specifically expressed in macrophages, promoters such as CD11b (Mac-1) may be used (Hickstein DD., Proc. Natl. Acad. Sci. USA, 89: 2105-2109, 1992).

Furthermore, promoter regions derived from chromosomal DNA may be obtained by cloning a chromosomal DNA library, treating with a restriction enzyme, and preparing DNA fragments having the promoter activity of interest. Alternatively, the DNA fragments of interest may be prepared by PCR using suitable primers with chromosomal DNA as a template.

Virus-derived promoters and enhancers can be obtained by RT-PCR or PCR using a virus genome or chromosomal DNA into which the DNA region of interest has been integrated.

As the promoter that expresses a prodrug ribozyme, a single gene-derived promoter may be used alone or an enhancer or a control region contained in a plurality of promoter regions may be used to construct new artificial promoters.

The promoter regions thus obtained may be ligated using a prodrug ribozyme gene having a poly(A) signal sequence 3' downstream and T4 DNA ligase. This enables the expression of the prodrug ribozyme gene.

Tissue-specific enhancers of the present invention are enhancers that exhibit an enhancer activity only in specific tissues. For example, enhancers of a gene that is specifically expressed in the liver such as the enhancer sequence of HBV (Aragona E. et al., J. Pathol., 180: 441-449, 1996) may be used.

By including a disease-specific promoter and/or enhancer into the expression vector of the present invention, prodrug ribozymes can be expressed for the purpose of treating specific diseases. For example, for virus infections for which no tissue-specific infectivity is observed, there can be used a promoter activated by a transcription factor produced by the virus itself in addition to a promoter of the gene that is universally expressed in the living body. It is also possible to use the promoter of the virus itself. Promoters of. the gene specifically expressed in diseases and of marker proteins may also be used. In this case, it is possible to combine one type of promoter or a plurality of promoters and enhancers or silencers.

By including, in the expression vector of the present invention, a sequence having the activity of activating the extranuclear transport of mRNA or of stabilizing mature mRNA, ribozyme molecules having an RNA-cleaving activity in the cytoplasm can be stably supplied. As used herein, "a sequence having an activity of activating the extranuclear transport of mRNA" means a sequence that binds to proteins in the cell by taking a unique structure, and, by so doing, facilitates the transport of mRNA from the nucleus to the cytoplasm. As the nuclear protein, there can be mentioned a protein that activates the transport of RNA from within the nucleus to outside the nucleus. "A sequence having an activity of stabilizing mature mRNA" as used herein means a sequence that is resistant to digestion by RNase, through which mature mRNA can be stabilized. As such sequences, there can be mentioned those shown below.

### A. Constitutive transport element (CTE) sequence

As the sequence that activates the extranuclear transport of ribozymes converted from prodrug ribozymes in the nucleus, there can be used CTE sequences found in monkey retrovirus such as MPMV, SRV-1, SRV-2 etc. (Tang, H. et al., Virology, 228: 333-330, 1997). It has been demonstrated that when a CTE sequence was inserted into an intervening sequence of pre-mRNA of adenovirus, extranuclear transport can be activated without affecting the efficiency of splicing (Saavedra C. et al., Curr. Biol., 7: 619-628, 1997).

### B. Zipcode region

As the sequence that activates the extranuclear transport of ribozymes converted from prodrug ribozymes in the nucleus, there can be used a zipcode region.

As the structure that facilitates the migration of mRNA from within the nucleus to the cytoplasm, there are known specific base sequences such as ACACCC as in mRNA of β-actin, in addition to the cap structure at the 5'-end of mRNA and a poly(A) sequence. It has been demonstrated that an RNA binding protein binds to this sequence, thereby resulting in enhanced stability of mRNA in the cytoplasm. More specifically, a zipcode-bindingprotein (ZBP-1) specifically binds to the zipcode region comprising 54 bases including the ACACCC sequence, transports mRNA from within the nucleus to the cytoplasm, and facilitates localization of mRNA in the cytoplasm (Kislauskis EH et al., J. Cell Biol., 127: 441-451, 1994, Ross AF et al., Mol. Cell. Biol., 17: 2158-2165, 1997).

There are many intervening sequences in the β-actin gene. But, the zipcode sequence is thought to be an extranuclear transport-activating region that is different from the CTE sequence in that only one molecular species was detected as mRNA. By ligating a binding sequence to which an RNA-binding protein such as this specifically binds to a prodrug ribozyme molecule, it is possible to stably supply a large quantity of mature ribozyme without molecules that undergo alternative splicing or prodrug ribozyme molecules being transported to the cytoplasm.

Specifically, by PCR using primers to which a restriction enzyme recognition site was added at the 5'-end that can amplify the zipcode region comprising 54 bases present immediately after the termination codon of the chicken β-actin gene, the DNA fragment of interest can be amplified. After cleaving with a restriction enzyme, this is inserted to upstream of the poly(A)-added signal of the vector that expresses prodrug ribozyme genes. Then, the prodrug ribozyme molecule expression vector into which the zipcode sequence has or has not been inserted is introduced into a cultured cell line. In the former, an increase in mature ribozymes in the cytoplasm may be confirmed by Northern hybridization using as the probe DNA encoding the mature ribozyme molecule, PCR or the like.

### C. Histone mRNA-stabilizing sequence

In the 3'-end of histone mRNA to which no poly(A) sequence is added, it has been demonstrated, a common 5'-GCYYYUUYUNARRRCC-3' sequence (Y represents C or U, R represents A or G, and N represents any base) (SEQ ID NO: 51) contributes to stabilizing mature histone mRNA (Gabb HA et al., J. Biomol. Struct. Dyn., 9, 1119-1130, 1992). By ligating such a histone mRNA-stabilizing sequence comprising 16 bases to a prodrug ribozyme molecule, it permits a stable supply of mature ribozymes in large quantities.

Specifically, a double-stranded DNA that corresponds to the 16 bases present at the 3'-end of the human histone gene is synthesized. At this time, it is desired to add a restriction enzyme recognition site to both ends of the synthesized DNA. The synthesized double-stranded DNA is inserted to downstream of the prodrug ribozyme gene of the vector that was cleaved with the desired restriction enzyme and that expresses the prodrug ribozyme gene.

Then, the prodrug ribozyme molecule expression vector into which histone mRNA-stabilizing sequence has or has not been inserted is introduced into a cultured cell line, and an increase in mature ribozyme in the cytoplasm may be confirmed by Northern hybridization using as the probe DNA encoding the mature ribozyme molecule, PCR or the like.

The third embodiment of the present invention is one or multiple (a plurality of) prodrug ribozyme having the following features:
(a) containing, in the ribozyme sequence, an intervening sequence that is eliminated by splicing, and
(b) having,no RNA-cleaving activity.

The fourth embodiment of the present invention is a pharmaceutical composition comprising as an active ingredient, the gene of the first embodiment of the present invention. The pharmaceutical composition of the present invention may be used as a therapeutic agent having the gene itself encoding the prodrug ribozyme of the first embodiment as an active ingredient, or may be used as gene therapy agent in which the expression vector of the second embodiment may be administered as an active ingredient to patients and allowed to be expressed in the body.

Diseases to which the pharmaceutical composition of the present invention may apply include, but not limited to, viral infections, cancer and diseases caused by excessive expression of specific genes or by the appearance of abnormal proteins. As the viral infections, there can be mentioned chronic diseases by HCV and HBV or human immune deficiency syndrome. As the cancer, there can be mentioned lung cancer, pancreatic cancer or colon cancer in which the mutation of ras gene is observed at high frequencies, or leukemia in which chromosome translocation is observed such as c-myc or abl gene. As diseases caused by excessive expression of specific genes or the appearance of abnormal proteins, there can be mentioned cardiovascular diseases, immunological diseases such as allergy and autoimmune diseases, neurotic diseases such as neural disorders, familial hereditary diseases, primary genetic diseases and sporadic genetic diseases, and the like. The pharmaceutical composition may also be applied to infection by bacteria, rickettsia, fungi, and the like.

The scope to which the pharmaceutical composition of the present invention is applied encompasses not only humans but mammals represented by monkeys, cattle, horses, sheep, goat, pigs, donkeys, chickens, dogs, cats, rats, mice, rabbits and guinea pigs, and plants.

The pharmaceutical composition of the present invention may be administered together with adjuvants or may be administered in a dosage form of particles. As the dosage form, more specifically, they may be formulated into liposome formulations, particulate formulations bound to beads with a diameter of several µm, formulations bound to lipids, and the like. a s the dosage form, there can be mentioned sustained-release minipellet formulations and the like. Furthermore, drugs are passed through the cell membrane, a peptide that enhances the efficiency of passing through the cell membrane may be mixed. The efficiency of passing through the cell membrane may also be enhanced by linking specific sugars. The dosage may be appropriately adjusted depending on the severity of disease, the age, weight etc. of the patient. It is usually in the range of 0.001 mg/kg/dose to 1000 mg/kg/dose, which is preferably administered every day during the starting period, and thereafter every several days to several months. As the dosage regimen, there can be mentioned oral administration, intraarterial injection, intravenous injection, intramuscular injection, and local injection.

When an agent for gene therapy comprising an expression vector, the second embodiment of the present invention, as an active ingredient is introduced into the cell, the dosage forms are roughly divided into two groups: an embodiment that employs non-viral vectors and an embodiment that employs viral vectors.

More specifically, as means of allowing a gene encoding a prodrug ribozyme to act in the cell, there can be mentioned the following methods:
A. When non-viral vectors are used, a gene encoding a prodrug ribozyme molecule is introduced into a recombinant expression vector, and then the recombinant expression vector may be incorporated into the cell by any of the calcium phosphate co-precipitation method, a method of introducing DNA molecules into liposomes (the liposome method, the lipofectin method, the lipofectamine method), the electroporation method, the microinjection method, the 'method of introducing DNA molecules with carriers into the cell using a particle gun, and the like. It is also possible to mix with a cationic polymer such as poly-L-lysine and poly-L-arginine or a peptide or polypeptide that activates nuclear migration such as HMG protein and histone protein, which are then introduced into the cell. Alternatively, in order to accumulate DNA molecules specifically in the liver, for example, peptides or polypeptides may be modified with asialoglycoprotein or galactose.
B. As viral vectors, representative, methods employ viral vectors such as adenovirus and retrovirus. More specifically, a gene encoding a prodrug ribozyme molecule is introduced into, for example, a DNA virus such as detoxified retrovirus, adenovirus, adeno-associated virus, herpesvirus, vaccinia'virus, poxvirus, poliovirus, Sindbis virus, Sendai virus, SV40, and human immunodeficiency virus (HIV), or an RNA virus to infect the cell with a recombinant virus, so that the gene is introduced into the cell.

By employing any of these methods, it is possible to introduce a gene encoding a prodrug ribozyme molecule into the cell. In gene therapy with non-viral vectors, it is desirable to bring the gene into the proximity of the disease site by a combination of local administration with a dosage form having an enhanced tissue migration. On the other hand, in gene therapy with viral vectors, local administration is not always required and intravenous administration may also be used.

When the agent for gene therapy of the present invention is used as a pharmaceutical drug, there are the in vivo method in which DNA is directly introduced into the body, and the ex vivo method in which certain cells are removed from a human and DNA is introduced into said cells ex vivo and the cell is returned to the body (Nikkei Science, Gekkann yakuji (Monthly Pharmaceutical Affairs), 36(1): 23-48, 1994; Jikkenn Igaku (Experimental Medicine), 12(15) 1994). In accordance with the present invention, the in vivo method is preferred.

For agents for gene therapy employing the recombinant adenovirus of the present invention, it is known that among the above viral vectors adenovirus has the highest efficiency of infection, and therefore the use of the adenovirus vector system is most preferred.

The fifth embodiment of the present invention is a method of producing mature ribozymes having an RNA-cleaving activity in the cytoplasm, said method comprising introducing a gene, the first embodiment of the present invention, into an eukaryotic cell.

The present invention will now be explained in further details hereinbelow with reference to Examples. It should be noted, however, that the present invention will not be limited by them in any way.

### Example 1

### Selection of a ribozyme target sequence against HCV NS5B

### (1) The + strand target sequence

For the purpose of creating a ribozyme that recognizes HCV regions and a prodrug ribozyme into which an intervening sequence has been inserted, homology search was conducted focusing on the regions highly conserved between HCV genotypes using a computer software (GeneWorks) by Teijin System Technology. As the HCV regions having high homology, there can be mentioned the core region, the RNA-dependent RNA polymerase region (NS5B) etc., but HGV that is an HCV-related virus lacks the core sequence. HGV exhibits co-infection with HCV, and the concurrent removal of HGV with a ribozyme was considered to have a higher therapeutic effect in the treatment of HCV diseases. Thus, homology was investigated for the RNA-dependent RNA polymerase region (NS5B) having a high homology, and as a result, target sequences for RNA cleavage by ribozyme were selected.

In the treatment of HCV, interferon has been used, but genotype 1b is known to be highly interferon-resistant. Thus, in this Example, the target sequence of ribozyme that can cleave HCV NS5B RNA was mainly examined. In order to target the base sequence of HCV having a high rate of mutation, G:U base pairing was taken into consideration in addition to G:C and A:U pairing.

Thirteen sequences of the NS5B region were selected mainly from HCV genotype 1b, and DNA homology analysis was performed on them using a software GeneWorks (Teijin System Technology) and a software DNASIS (Hitachi Software Engineering). Sequences were selected that meet the conditions of sequences needed for cleaving ribozyme and the boundary sequences thereof have high homology. The 13 sequences selected in the HCV NS5B region were the sequences of Accession No. X61596, D90208, D11168, D85516, M58335, D50480, D50481, D50482, D50483, D50484, and D50485 as HCV subtype 1b, the sequence of Accession No. D00944 as HCV subtype 2b, and the sequence of Accession No. D10988.

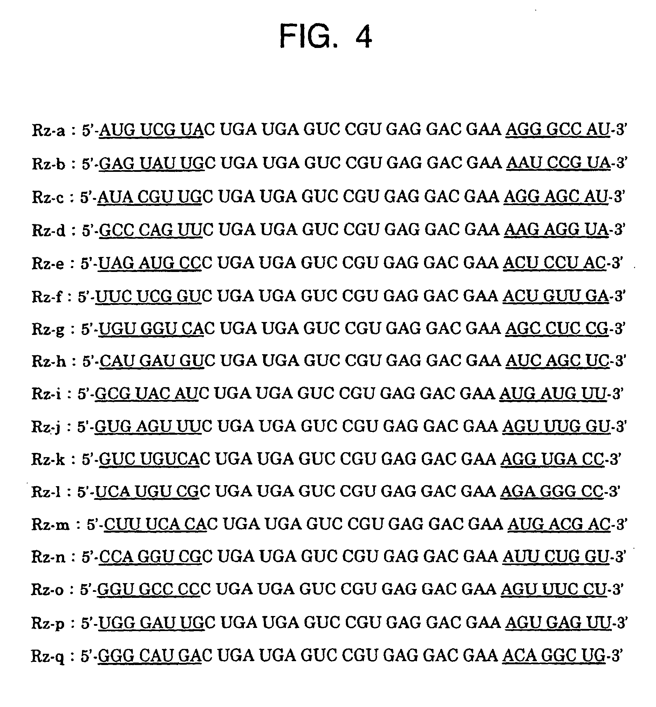

As a result of the analysis, 17 types of + strand target sequences were selected from the regions that are highly conserved and that can be cleaved on the base sequences. Thereafter, ribozymes to respective target sequences were set up and the stability of the structure that has a cleaving activity of ribozyme was investigated using a software Oligo by National Biosciences. The base sequences of the set up 17 ribozyme molecules (in an alphabetical order: Rz-a to Rz-q, SEQ ID NO: 19 to 35) are shown in Figure 4. The target sequences in HCV NS5B + strand RNA (SEQ ID NO: 1) that is a substrate estimated to be cleavable with these ribozyme molecules (in an alphabetical order: Rz-a to Rz-q) are shown in SEQ ID NO: 2 to 18 in this order.

### (2) The - strand target sequences

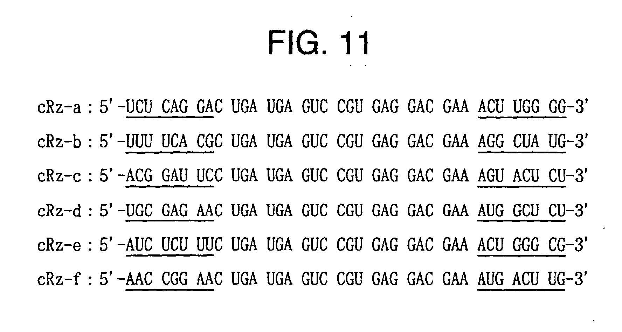

In a manner similar to the method used for the selection of HCV NS5B + strand target sequences, six target sequences in. the HCV NS5B - strand were selected. Thereafter, ribozymes to respective target sequences were set up and the stability of the structure having a cleaving activity of ribozyme was investigated using a software Oligo by National Biosciences. The base sequences of the six ribozyme molecules (in an alphabetical order: cRz-a to cRz-f, SEQ ID NO: 64 to 69) set up are shown in Figure 11. The - strand target sequences in HCV NS5B RNA that is a substrate estimated to be cleavable with these ribozyme molecules (in an alphabetical order: cRz-a to cRz-f) are shown in SEQ ID NO: 58 to 63 in this order.

### Example 2.

### Evaluation of the in vitro cleaving activity of ribozymes to HCV NS5B

### (1) The + strand target sequence

Hammerhead type ribozyme molecules (Rz-a to Rz-q) were prepared by RNA synthesis, and were investigated whether they have an in vitro cleaving activity. Ribozyme RNA was chemically synthesized and was purified by HPLC.

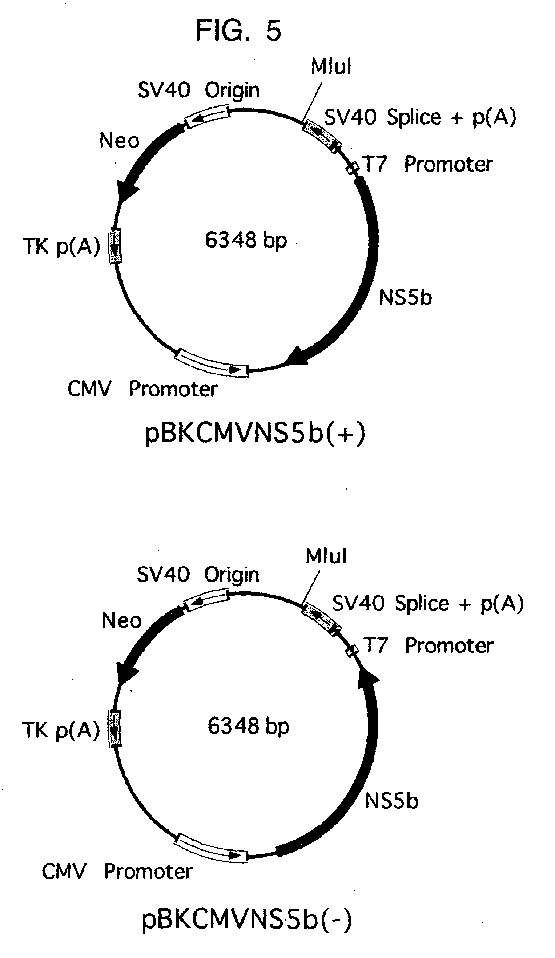

In order to prepare the substrate, plasmid pBKCMVNS5B(+) in which the HCV NS5B gene (prepared from pUCHCV1 donated by Dr. Norio Hayashi of Osaka University) derived from an HCV hepatitis patient was inserted in the forward direction into a HindIII cleavage site downstream to T7 promoter of plasmid vector pBKCMV and plasmid pBKCMVNS5B(-) in which the same was inserted in the opposite direction were constructed. pBKCMVNS5B(+) and pBKCMVNS5B(-) are shown in Figure 5. The base sequence of the inserted HCV NS5B region is shown in SEQ ID NO: 1.

The substrate NS5B RNA was prepared by an in vitro transcription reaction from a recombinant plasmid pBKCMVNS5B(+) in which NS5B DNA was introduced into pBK-CMV. Thus, after the both plasmids were digested with a restriction enzyme EcoRI, RNase was inactivated with Proteinase K and purified. Then, using an in vitro transcription kit (Ambion), a transcription reaction was performed in vitro by T7 RNA polymerase with the above plasmid as a template, whereupon the substrate was labeled with [α-³²P]UTP. After treatment with DNase, RNA was precipitated and dissolved in a RNase-free sterile water, and the concentration was determined using a spectrophotometer. As a result, a labeled NS5B RNA comprising about 1.8 Kb was obtained. The labeled NS5B RNA and a ribozyme molecule that has a typical backbone obtained by the above RNA synthesis were mixed, and the in vitro cleaving activity was evaluated. In a solution containing 50 mM Tris-HCl (pH 7.5) and 20 mM MgCl₂, 50 pmole of the ribozyme and 0.05 pmole of the substrate RNA were mixed, which were cleaved at 37°C for four hours, and the reaction was stopped by adding EDTA to a final concentration of 50 mM. Cleavage of the substrate RNA was confirmed by adding an equal amount of electrophoresis buffer containing 50 mM EDTA and 95% formamide to the reaction mixture, which was subjected to autoradiography analysis.

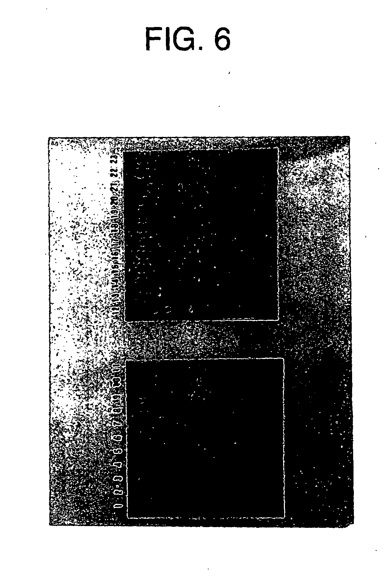

As a result, 9 ribozyme molecules were found to have cleaving activity. Figure 6 shows the result of investigation of the cleaving activity to the substrate. It was indicated that for 9 ribozymes a full-length NS5B RNA was cleaved at the target site, judged from the length of each of two newly created RNA molecules. Among the 9 ribozymes, stronger cleaving activity was observed for Rz-a (the target sequence is described in SEQ ID NO: 2), Rz-n (the target sequence is described in SEQ ID NO: 15), and Rz-p (the target sequence is described in SEQ ID NO: 17) compared to Rz-b (the target sequence is described in SEQ ID NO: 3), Rz-f (the target sequence is described in SEQ ID NO: 7), Rz-i (the target sequence is described in SEQ ID NO: 10), Rz-1 (the target sequence is described in SEQ ID NO: 13), Rz-m (the target sequence is described in SEQ ID NO: 14), and Rz-o (the target sequence is described in SEQ ID NO: 16). No cleaving activity was observed for the rest of 8 ribozymes Rz-c (the target sequence is described in SEQ ID NO: 4), Rz-d (the target sequence is described in SEQ ID NO: 5), Rz-e (the target sequence is described in SEQ ID NO: 6), Rz-g (the target sequence is described in SEQ ID NO: 8), Rz-h (the target sequence is described in SEQ ID NO: 9), Rz-j (the target sequence is described in SEQ ID NO: 11), Rz-k (the target sequence is described in SEQ ID NO: 12), and Rz-q (the target sequence is described in SEQ ID NO: 18). These target sequences for which no cleavage was observed were mostly present at sites that are estimated to have a stem structure in the secondary structure of NS5B RNA obtained using a computer software (DNASIS) by Hitachi Software Engineering..

For Rz-a that has no cleaving activity for the - strand, cleavage specificity for the + strand is shown.

The target sequences (SEQ ID NO: 42-50) for the 9 cleavable ribozymes (Rz-a, Rz-b, Rz-f, Rz-i, Rz-1, Rz-m, Rz-n, Rz-o, Rz-p) are as follows. Y in the sequences represents C or U, R represents A or G, V represents A or C or G, B represents C or G or U, M represents A or C, W represents A or U, and S represents C or G.

1: AUGGCYCUYU AYGAYRU

2: UAYGGRUUCC ARUACUC

3: UCAACVGUCA CBGAGAR
4: AAYAUYAUYM WGUWYGC
5: GGCYCUYUAY GAYRUSR
6: GUYGUYAUCU SWGARAG
7: ACCRGAAUAY GACYUGG
8: AGRAAACUUG GRGYVCC
9: AACUCACUCC AWUBCCV

Among the 9 cleavable ribozymes, Rz-a, Rz-b and Rz-n were thought to cleave all of the 13 HCV NS5B RNAs described in Example 1. The rest six ribozymes were also thought to cleave all of 11 1b type HCV RNAs described in Example 1.

HCV is a virus that undergoes genetic mutation at high frequency between different genotypes and between the same genotypes, and thus, when mutation takes place in 1 to several bases in the above sequences, it is likely to become sequences that cannot be recognized by ribozyme molecules. However, these target sequences are believed to be present at sites of the secondary structure of RNA that is easily recognized by ribozyme molecules. Therefore, for those HCV that had a substitution of several bases that does not significantly affect the secondary structure of RNA, ribozyme molecules that correspond to the mutated sequences could cleave HCV NS5B RNA.

### (2) The - strand target sequences

The substrate RNA to be used for the investigation of cleaving activity of ribozymes that cleave the HCV NS5B - strand was prepared as follows:

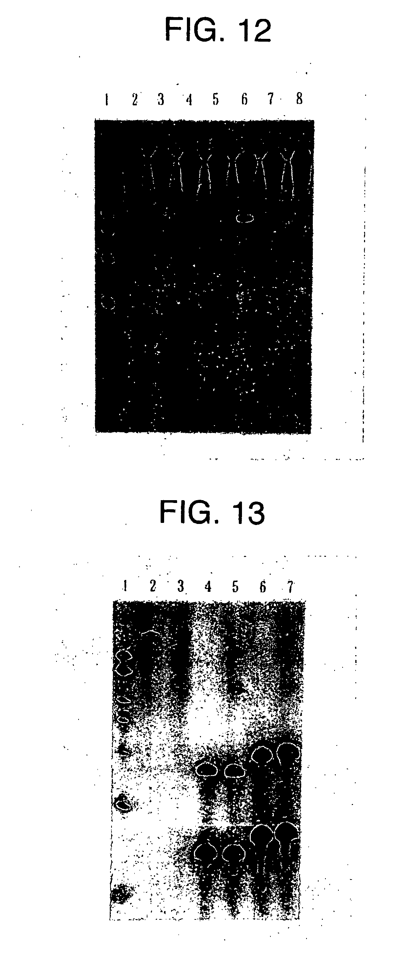

After pBKCMVNSSB(-) was digested with a restriction enzyme EcoRI, RNase was inactivated with Proteinase K and purified. Then, using an in vitro transcription kit (Ambion), a transcription reaction was performed in vitro by T7 RNA polymerase, whereupon the substrate was labeled with [α-³²P]UTP. After treatment with DNase, RNA was precipitated and dissolved in a RNase-free sterile water, and the concentration was determined using a spectrophotometer. The cleaving activity of ribozymes was evaluated as follows. Thus, 0.05 pmole of the NS5B - strand RNA and 50 pmole of the each of synthesized ribozymes were mixed, and were reacted in a solution containing 50 mM Tris-HCl (pH 7.5) and 20 mM MgCl₂. After allowing to react at 37°C for two hours, the reaction was stopped by adding EDTA to a final concentration of 50 mM. Cleavage was analyzed by adding the electrophoresis buffer containing 50 mM EDTA and 95% formamide to the reaction mixture, which was subjected to denatured polyacrylamide gel electrophoresis and then analyzed by autoradiography. As a result, among six ribozymes, five ribozymes (cRz-a, cRz-b, cRz-d, cRz-e, cRz-f) were found to have a cleaving activity (Figure 12).

The target sequences (SEQ ID NO: 70-74) of the five cleavable ribozymes (cRz-a, cRz-b, cRz-d, cRz-e, cRz-f) are as follows. Y in the sequences represents C or U, R represents A or G, H represents A or C or U.

1: CYCCAAGUHU YCUGAGR
2: CAURGCYUHC GUGAARR
3: AGRGCCAUYU UYUCGCA
4: YGCCCAGUUR AAGAGRU
5: CARGUCRUHU UCYGGUU

### Example 3

### Evaluation of in vitro cleaving activity of ribozymes having a restriction enzyme NotI recognition sequence

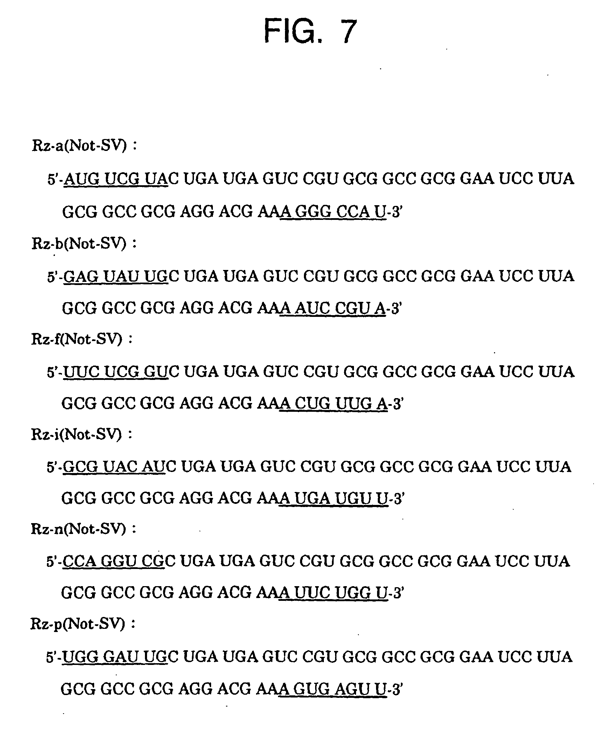

For ribozymes having, in the stem structure, a restriction enzyme NotI sequence for inserting an intervening sequence and having, in the loop structure, a base sequence containing a spacer comprising a five base junction site formed when the intervening sequence in the gene expressed from a SV40 late promoter is removed, the cleaving activity is evaluated before the insertion of intervening sequences. The typical sequences of six ribozyme molecules (SEQ ID NO: 36-41) to be prepared are shown in Figure 7.

The procedure for the preparation is as follows:
1) After two oligonucleotides comprising 70 bases encoding a ribozyme molecule are chemically synthesized, they are purified by HPLC, the 5'-ends are phosophrylated and then annealed. For-example, in the case of Rz-a Rz-a-Not-upper (5'-AGCTTATGTCGTACTGATGAGTCCGTGCGGCCGCGGAATCCTTAGCGGCCGCGAG GACGAAAGGGCCATA-3') (SEQ ID NO: 52), and Rz-a-Not-lower (5'-AGCTTATGGCCCTTTCGTCCTCGCGGCCGCTAAGGATTCCGCGGCCGCACGGACT CATCAGTACGACATA-3')(SEQ ID NO: 53) are prepared, as oligonucleotides applied to construct a mature Rz-a ribozyme after elimination of the intervening sequence in a living body. In the case of Rz-b, Rz-f, Rz-i, Rz-n, or Rz-p, the construction is employed the same means.
   Using a ligation reaction, amended fragment is integrated into a suitable restriction enzyme cleavage site of pcDNA3.1(+) in which mutation was caused in a NotI cleavage site in the vector by Klenow fragment or T4 DNA polymerase. For the ligation reaction, DNA Ligation Kit Ver. 2 of Takara Shuzo is used. Then, it is transformed into JM109 E. coli competent cells and allow colonies to appear. Plasmid DNA is prepared from the clones obtained, cleaved with a suitable restriction enzyme thereby to confirm that the fragment of the desired length has been inserted.
2) In order to confirm the direction of insertion, base sequences are analyzed using the primer of the vector (T7 promoter primer or pcDNA3.1/BGH reverse primer). The sequences of the primers used are 5'-TAATACGACTCACTATAGGG-3' (T7 promoter primer, SEQ ID NO: 54), 5'-TAGAAGGCACAGTCGAGG-3' (pcDNA3.1(+)/BGH reverse primer, SEQ ID NO: 55). The sequencing kit by Perkin-Elmer is used'for the sequence reaction, and the sequencer by Applied Biosystems is used for the analysis of base sequences. The plasmids of interest thus obtained are designated pcDNARz-a(m), pcDNARz-b(m), pcDNARz-f(m), pcDNARz-i(m), pcDNARz-n(m), and pcDNARz-p(m).
3) After pcDNARz-a(m), pcDNARz-b(m), pcDNARz-f(m), pcDNARz-i(m), pcDNARz-n(m), and pcDNARz-p(m) are prepared, they are digested with a suitable restriction enzyme and purified, and then ribozyme molecules are prepared using an in vitro transcription kit by Ambion. These molecules are those that can be transcribed by T7 RNA polymerase.
4) Using ³²P-labeled RNA prepared by the in vitro transcription reaction from recombinant plasmid pBKCMVNS5B(+) as the substrate, and adding the above mature ribozyme molecules, the cleaving activity is investigated.

In this method, six mature ribozymes Rz-a (Not-SV), Rz-b (Not-SV), Rz-f (Not-SV), Rz-i (Not-SV), Rz-n (Not-SV), and Rz-p (Not-SV) shown in Figure 7 are confirmed to have an equal activity to that of the ribozymes described in Figure 4, and it is confirmed that ribozymes after elimination of intervening sequences have the ability to cleave the substrate RNA.

In fact, it was investigated whether Rz-a (Not-SV) has an ability to cleave the target sequence. In order to synthesize plasmid that expresses Rz-a (Not-SV) containing a NotI sequence in the stem structure region of ribozyme and, as a control, plasmid that expresses Rz-a not containing a NotI sequence, two oligonucleotides encoding these ribozyme molecules were chemically synthesized and purified. After phosphorylating the 5'-ends and annealing, they were inserted into restriction enzymes NheI and HindIII sites of pcDNA3.1(+) that underwent mutation at a NotI cleavage site. These ribozyme expression vectors were digested with a restriction enzyme XbaI, and purified after inactivation of RNase by Proteinase K. Then, using an in vitro transcription kit (Ambion), a transcription reaction was performed in vitro by T7 RNA polymerase, whereupon the substrate was labeled with [α-³²P)UTP. After treatment with DNase; RNA was precipitated and added to an electrophoresis buffer containing 95% formamide to perform electrophoresis in denatured acrylamide gel. After excising ribozyme fragments and purifying them, in vitro transcribed ribozyme RNA was obtained.

pBKCMVNS5B(+) was digested with a restriction enzyme EcoRI, and purified after inactivation of RNase by Proteinase K. Then, using an in vitro transcription kit (Ambion), a transcription reaction was performed in vitro by T7 RNA polymerase, whereupon the substrate was labeled with [α-³²P]UTP. After treatment with DNase, RNA was precipitated and dissolved in a RNase-free sterile water, and the concentration was determined using a spectrophotometer. Thus, the NS5B + strand RNA was obtained.

After 0.05 pmole of the NS5B + strand RNA and ribozymes were mixed and were reacted in a solution containing 50 mM Tris-HCl (pH 7.5) and 20 mM MgCl₂ at 37°C for four hours, the reaction was stopped by adding EDTA to a final concentration of 50 mM. An equal amount of electrophoresis buffer containing 50 mM EDTA at a final concentration and 95% formamide was added, which was subjected to denatured polyacrylamide gel electrophoresis, and then analyzed by autoradiography.

As a result, ribozymes containing a restriction enzyme recognition sequence in the stem structure of the ribozyme were found to have a cleaving activity (Figure 13).

Furthermore, in order to evaluate whether ribozyme molecules cleave mRNA other than the target molecule derived from the cell, human liver-derived mRNA purchased form Clontech was added stepwise to"the in vitro reaction mixture, and then the limiting cleaving activity to labeled NS5B RNA can be investigated.

### Example 4

### Insertion of an intervening sequence into ribozymes

After cleaving respective recombinant plasmid DNAs containing six mature ribozymes selected by the method described in Example 3, an intervening sequence is inserted.

The procedure for the insertion of an intervening sequence is as follows:
1) After two oligonucleotides comprising 83 bases corresponding to the SV40 small T intron region are chemically synthesized, they are purified by HPLC, the 5'-ends are phosophrylated and then annealed. The oligonucleotides used are SV40 samll T intron-lower (5'-GGCCGCGGAATCTAAAATACACAAACAATTAGAATCAGTAGTTTAACACATTATA CACTTAAAAATTTTATATTTACCTTAGC-3')(SEQ ID NO: 56), and SV40 samll T intron-upper (5'-GGCCGCTAAGGTAAATATAAAATTTTTAAGTGTATAATGTGTTAAACTACTGATT CTAATTGTTTGTGTATTTTAGATTCCGC-3') (SEQ ID NO: 57).
   After digesting pcDNA3.1(+) (pcDNARz-a(m) etc.) containing a ribozyme with NotI, it is dephosphorylated with alkaline phosphatase. This vector and the above annealed oligonucleotide are ligated. For the ligation reaction, DNA Ligation Kit Ver. 2 of Takara Shuzo is used. Then, it is transformed into JM109 E. coli competent cells and allow colonies to appear. Plasmid DNA is prepared from the clones obtained, cleaved with a suitable restriction enzyme thereby to confirm that the fragment of the desired length has been inserted.
2) In order to confirm the direction of insertion, the base sequence is analyzed using the primer of the vector (T7 promoter primer or pcDNA3.1/BGH reverse primer). The sequencing kit by Perkin-Elmer is used for the sequence reaction, and the sequenser by Applied Biosystems is used for the analysis of base sequences. The plasmids of interest thus obtained are designated pcDNARz-a(i), pcDNARz-b(i), pcDNARz-f(i), pcDNARz-i(i), pcDNARz-n(i), and pcDNARz-p(i).

By an in vitro transcription reaction using these plasmids, prodrug ribozymes containing an intervening sequence are prepared, and the absence of an in vitro RNA-cleaving activity in these prodrug ribozymes can be confirmed in a similar manner to Example 2.

In order to confirm that prodrug ribozyme molecules have become mature ribozyme molecules in which intervening sequences have been eliminated in the cell, the DNA-calcium phosphate co-precipitation method is used to perform DNA transfection on human kidney-derived strain COS1 cells and human liver-derived strain HepG2. One day after the transfection of the plasmid into the cell, 200 µg/ml neomycin is added to the medium. After culturing for 2 weeks, cells having the plasmid DNA are selected. From the cells obtained, total RNA in the cytoplasm is collected using the total RNA purification kit (ISOGEN) of Nippon Gene, and is subjected to Northern hybridization using mature ribozyme gene DNA as a probe. DNA probing is performed using T4 DNA kinase and [γ-³²P]ATP at the 5'-end. The hybridization condition is as follows: the reaction is performed in 0.5 M Na₂HPO₄ (pH 7.2), 7% SDS, and 1 mM EDTA solution (65°C) containing the probe, and a filter is washed in 40 mM Na₂HPO₄ (pH 7.2) and 1% SDS solution. By this method, it can be confirmed that prodrug ribozyme molecules are expressed from recombinant vectors expressing prodrug ribozyme genes, and that mature molecules in which intervening sequences have been eliminated by splicing are expressed.

### Example 5

### Construction of cultured cell lines expressing NS5B RNA of HCV

In order to evaluate the cleaving activity to NS5B RNA of HCV in the cell, cultured cell lines. expressing NS5B RNA of HCV were constructed. The procedure for the construction is as follows:

pBKCMVNS5B(+) or pBKCMVNS5B(-) (see Figure 5) expressing NS5B RNA is introduced (4 µg and 1 µg each of DNA) by the calcium phosphate method (Promega) and the Transorm kit (Wako Pure Chemicals Industries, Ltd.) into 6 x 105 COS1 cells and 4 × 10⁵ HepG2 cells per 21 cm2 dish. Two days after the introduction of the gene, 500 µg/ml and 1000 µg/ml each of neomycin were added, and culturing was continued for 14-17 days. Then 48 and 59 clones each having drug resistance were selected. The efficiency of transformation in each introduction experiment is shown in Table 1.

**Table 1**

| | | |
|---|---|---|
| COS-1 cells | pBKCMVNS5B(-)/Linearized at MluI Site | |
| | Calcium phosphate method | 0.2% |
| | Lipofection method (Transorm) | 0.2% |
| | pBKCMVNS5B(-) /Circular | |
| | Calcium phosphate method | 0.06% |
| | Lipofection method (Transorm) | 0.3% |
| | pBKCMVNS5B(+)/Circular | |
| | Calcium phosphate method | 0.09% |
| | Lipofection method (Transorm) | 0.3% |
| HepG2 cells | pBKCMVNS5B(-)/Linearized at MluI Site | |
| | Calcium phosphate method | 0.06% |
| | Lipofection method (Transorm) | 0.05% |
| | pBKCMVNS5B(-)/Circular | |
| | Calcium phosphate method | 0.02% |
| | Lipofection method (Transorm) | 0.03% |
| | pBKCMVNS5B(+)/Circular /Circular | |
| | Calcium phosphate method | 0.03% |
| | Lipofection method (Transorm) | 0.06% |

From each of the selected clones, total RNA was prepared using ISOGEN of Nippon Gene, and the expression of NS5B was investigated by RT-PCR using the total RNA as a template.

Subsequently, Northern hybridization using, as a probe, total RNA of the clone groups in which NS5B was expressed was performed. For the preparation of probes, labeling with [α-³²P]dCTP was conducted using the Multiprime labeling kit of Amersham. In this method, transformants exhibiting a relatively high expression were selected.

### Example 6

### RNA-cleaving activity by ribozymes in cultured cells

To COS1 transformed cells or HepG2 transformed cells expressing HCV NS5B RNA, six prodrug ribozyme gene expression vectors constructed in the method of Example 4 are introduced to investigate the cleaving activity to NS5B RNA. The methods of introduction into cells and of preparation of total RNA are similar to that in Example 4. The cleaving activity by mature ribozymes are evaluated by Northern hybridization using the NS5B region as a probe as described above, PCR or the like. By this method, it can be confirmed that prodrug ribozyme molecules are expressed from recombinant vectors expressing six prodrug ribozyme genes, and that mature molecules in which intervening sequences have been eliminated by splicing retain the cleaving activity to HCV NS5B RNA in the cell.

This confirmation indicates that the target sequences of ribozymes to HCV NS5B RNA are useful sequences for the treatment of HCV diseases, and that methods of treating HCV diseases using prodrug ribozyme molecules are feasible.

### Example 7

### Effects of composite ribozymes

In order to elucidate the effects of composite type ribozymes in which two to several prodrug ribozymes that recognize HCV regions are ligated, a plurality of recombinant plasmid DNAs from six recombinant plasmid DNA expressing prodrug ribozyme genes are introduced into HepG2 transformed cells expressing HCV NS5B RNA. Then by showing that there is a significant decrease in the content of the remaining NS5B RNA, similar effects to when prodrug ribozyme genes are ligated can be indicated.

Thus, from among pcDNARz-a(i), pcDNARz-b(i), pcDNARz-f(i), pcDNARz-i(i), pcDNARz-n(i), and pcDNARz-p(i), two or more ribozymes are arbitrarily selected, and, at the same time, DNA transfection is performed to NS5B + strand RNA-expressing cells. The methods of introduction into cells and of preparation of total RNA are similar to those in Example 4. The cleaving activity by mature ribozymes are evaluated by Northern hybridization using the NS5B region as a probe as described above, PCR or the like. By this method, it can be confirmed that when two or more recombinant expression vectors are simultaneously introduced, the cleaving activity to HCV NS5B + strand RNA is higher than the introduction of a recombinant vector expressing one prodrug ribozyme gene. This confirmation clearly indicates that a plurality of mature molecule in which intervening sequences have been eliminated by splicing after expressing a plurality of prodrug ribozyme molecules retain strong cleaving activity to HCV NS5B + strand RNA in the cell, and, at the same time, indicates that they have a universal cleaving activity to HCV that easily undergoes genetic mutation.

The decrease in the content of remaining substrate RNA that was unexcised by cleavage with a plurality of ribozymes in the NS5B RNA-expressing transformed cells can be investigated more simply by using the substrate RNA synthesized in an in vitro transcription reaction.

Specifically, pBKCMVNS5B(+) was digested with a restriction enzyme EcoRI, and purified after inactivation of RNase by proteinase K. Then, using an in vitro transcription kit (Ambion), a transcription reaction was performed in vitro by T7 RNA polymerase, whereupon the substrate was labeled with [α-³²P]UTP. After treatment with DNase, RNA was precipitated and dissolved in a RNase-free sterile water, the concentration was determined using a spectrophotometer, and then NS5B + strand RNA was obtained.

0.05 pmole of the NS5B + strand RNA and 50 pmole of the single ribozyme or a mixture of 50 pmole each of Rz-a, Rz-b, and Rz-n were mixed, and the mixture was reacted at 37°C for two hours. The reaction was stopped by adding EDTA to a final concentration of 50 mM, which was added to an electrophoresis buffer containing 50 mM EDTA and 95% formamide. This was subjected to denatured polyacrylamide gel electrophoresis and then analyzed by autoradiography.

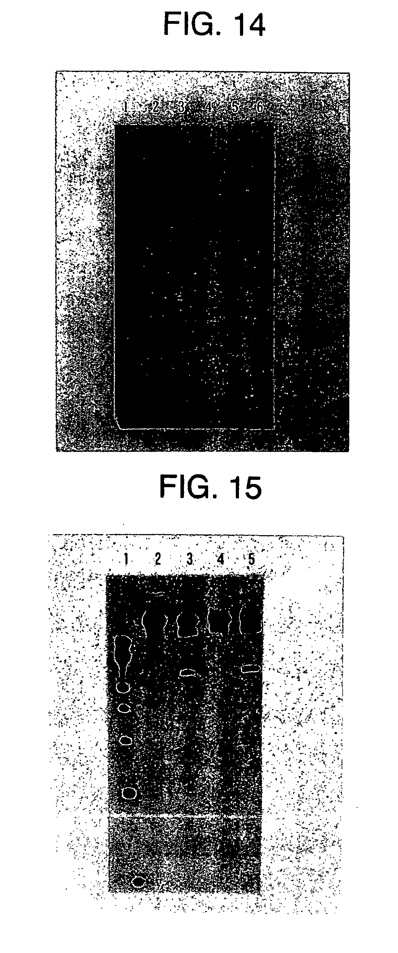

Figure 14 shows the result of the investigation on cleaving activity of a single ribozyme and mixtured ribozymes to the substrate NS5B + strand RNA. As shown in Figure 14, the substrate RNA can more easily be cleaved by a plurality of ribozymes than by a single ribozyme.

For therapeutic agents for HCV diseases by composite ribozymes comprising two to several contiguous ribozymes and prodrug ribozymes recognizing HCV regions, gene therapy by non-viral vectors as described above, recombinant plasmid DNA itself, that expresses one to several contiguous prodrug ribozyme genes retaining a strong cleaving activity to HCV NS5B RNA is believed to exhibit excellent therapeutic effects against acute HCV hepatitis or chronic HCV hepatitis.

Furthermore, it is also possible to express one to several contiguous prodrug ribozyme genes by a viral vector such as adenovirus vector, and this is also expected to exhibit excellent therapeutic effects.

### Example 8

### Evaluation of the in vitro cleaving activity of mature ribozymes containing no intervening sequences

Based on the typical structure of the ribozyme shown in Figure 10, Rz-a(-)S4 that was made to create homology with an adjacent region of the SV40 small T intervening sequence was chemically synthesized. Furthermore, based on the sequence of the adjacent region of the intervening sequence of SV40 small T, ribozyme Rz-a(-)S3 that was altered according to the general sequence of splicing was chemically synthesized.

Rz-a(-)S3 (SEQ ID NO: 75)
5'-AUG UCG UAC UGA AGA GGC AAG GAU GCC GAA AGG GCC AU-
3'

Rz-a(-)S4 (SEQ ID NO: 76)
5'-AUG UCG UAC UGA UGA GGA AUA AGG AUU CCG AAA GGG CCA
U-3'

After pBKCMVNS5B(+) was digested with a restriction enzyme EcoRI, and purified after inactivation of RNase by Proteinase K. Then, using an in vitro transcription kit (Ambion), a transcription reaction was performed in vitro by T7 RNA polymerase, whereupon the substrate was labeled with [α-³²P]UTP. After treatment with DNase, RNA was precipitated and dissolved in a RNase-free sterile water, the concentration was determined using a spectrophotometer, and NS5B + strand RNA was obtained.

0.05 pmole of the NS5B + strand RNA and 50 pmole of the ribozyme were mixed, and the mixture was reacted in a solution containing 50 mM Tris-HCl (pH 7.5) and 20 mM MgCl₂ at 37°C for two hours. The reaction was stopped by adding EDTA to a final concentration of 50 mM. An equal amount of an electrophoresis buffer containing 50 mM EDTA at a final concentration and 95% formamide was added. This was subjected to polyacrylamide gel electrophoresis and then analyzed by autoradiography.

As a result, both of ribozymes Rz-a(-)S3 and Rz-a(-)S4 were shown to have a cleaving activity. The result is shown in Figure 15.

### Example 9

### Evaluation of the in vitro cleaving activity of ribozymes containing a restriction enzyme EcoRII recognition sequence

The following ribozyme Rz-a(-)S6 (SEQ ID NO: 77) containing a restriction enzyme EcoRII recognition sequence and showing a profile that enables insertion of an intervening sequence into the site ⑤ shown in Figure 9 was chemically synthesized.

5'-AUG UCG UAC UGA AGA GAC CAG GAG GUC GAA AGG GCC AU-
3'

After pBKCMVNS5B(+) was digested with a restriction enzyme EcoRI, and purified after inactivation of RNase by Proteinase K. Then, using an in vitro transcription kit (Ambion), a transcription reaction was performed in vitro by T7 RNA polymerase, whereupon the substrate was labeled with [α-³²P]UTP. After treatment with DNase, RNA was precipitated and dissolved in a RNase-free sterile water, the concentration was determined using a spectrophotometer, and NS5B + strand RNA was obtained.

0.05 pmole of the NS5B + strand RNA and 50 pmole of the ribozyme were mixed, and the mixture was reacted in a solution containing 50 mM Tris-HCl (pH 7.5) and 20 mM MgCl₂ at 37°C for two hours. The reaction was stopped by adding EDTA to a final concentration of 50 mM. An equal amount of an electrophoresis buffer containing 50 mM EDTA at a final concentration and 95% formamide was added. This was subjected to denatured polyacrylamide gel electrophoresis and then analyzed by autoradiography.

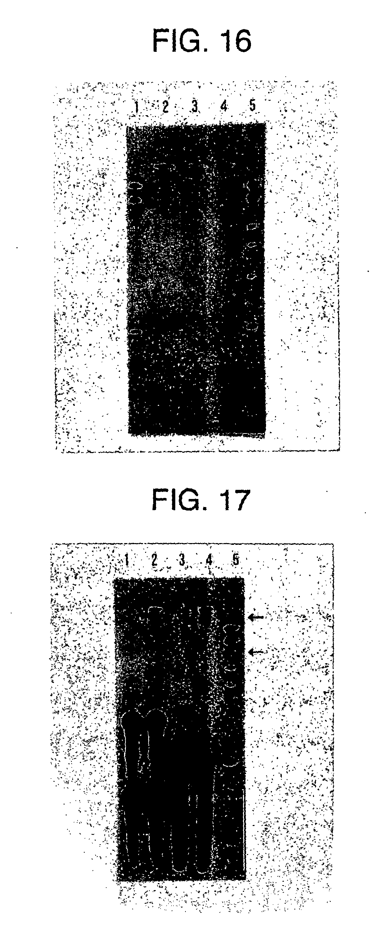

As a result, ribozymes containing a restriction enzyme EcoRII recognition sequence were shown to have a cleaving activity. The result is shown in Figure 16.

### Example 10

### Evaluation of the in vitro cleaving activity of ribozymes containing an intervening sequence in the loop

When an intervening sequence is introduced in the loop of a ribozyme, the loop region becomes large in length and is expected to become unstable. The difficulty of formation of the ribozyme structure causes the attenuation of cleaving activity. Thus, two ribozyme molecules of different length were created and the cleaving activity thereof was compared.

Rz-aS containing a SV40-derived intervening sequence in the loop structure site of ribozyme Rz-a, and Rz-a(-)S containing only a 5 base spacer sequence of the both ends of SV40-derived sequences contained in Rz-aS, were prepared.

Rz-aS (SEQ ID NO: 78)
5'-AUG UCG UAC UGA UGA GUC CGU GGA AUC UAA AAU ACA CAA
ACA AUU AGA AUC AGU AGU UUA ACA CAU UAU ACA CUU AAA AAU
UUU AUA UUU ACC UUA GAG GAC GAA AGG GCC AU-3'

Rz-a(-)S (SEQ ID NO: 79)
5'-AUG UCG UAC UGA UGA GUC CGU GGA AUC CUU AGA GGA CGA
AAG GGC CAU-3'

Plasmid pcΔN Rz-aS and pcΔN Rz-a(-)S that express Rz-a and Rz-a(-)S were constructed in the following manner.

Using, as a primer pair, two oligonucleotides containing a ribozyme sequence, a partial sequence of SV40 and a restriction enzyme NheI or HindIII recognition sequence at each 5'-end, PCR was performed with SV40 DNA (GIBCO) as a template. Then, the product was cleaved with restriction enzymes NheI and HindIII, a fragment was purified, which was inserted into restriction enzyme NheI and HindIII sites of a NotI cleavage site mutant pcDNA3.1(+) to construct pcΔN Rz-aS. The two oligonucleotides used are shown below:

Rz-aS(upper) (SEQ ID NO: 80)

5'-ACC CAA GCT GGC TAG CAT GTC GTA CTG ATG AGT CCG TGG
AAT CTA AAA ATA CAC AAA CAA TTA GAA TC-3'

Rz-aS(lower) (SEQ ID NO: 81)
5'-GCT CGG TAC CAA GCT TAT GGC CCT TTC GTC CTC TAA GGT
AAA TAT AAA ATT TTT AAG TGT ATA-3'

For pcΔN Rz-a(-)S, the forward strand and the reverse strand oligonucleotides encoding Rz-a(-)S were chemically synthesized and then pufiried, and their 5'ends were phosphorylated. After the both strands were annealed, they were inserted into restriction enzyme NheI and HindIII sites of a NotI cleavage site mutant pcDNA3.1(+). The two oligonucleotides used are shown below:

Rz-a(-)S (upper) (SEQ ID NO: 82)
5'-CTA GCA TGT CGT ACT GAT GAG TCC GTG GAA TCC TTA GAG
GAC GAA AGG GCC ATA-3'

Rz-a(-)S (lower) (SEQ ID NO: 83)
5'-AGC TTA TGG CCC TTT CGT CCT CTA AGG ATT CCA CGG ACT
CAT CAG TAC GAC ATG-3'

After these ribozyme expression vectors were digested with a restriction enzyme XbaI, and purified after inactivation of RNase by Proteinase K. Then, using an in vitro transcription kit (Ambion), a transcription reaction was performed in vitro by T7 RNA polymerase, whereupon the substrate was labeled with [α-³²P]UTP. After DNase treatment, protein was inactivated with phenol/chloroform and was removed. RNA was precipitated and then an electrophoresis buffer containing 95% formamide was added thereto. This was subjected to denatured acrylamide gel electrophoresis. Cleaved ribozymes were excised and purified, and then synthetic ribozymes were obtained by in vitro transcription. The amount of RNA was also calculated by determining its specific activity with a liquid scintillation counter.

After pBKCMVNS5B(+) was digested with a restriction enzyme EcoRI, and purified after inactivation of RNase by Proteinase K. Then, using an in vitro transcription kit (Ambion), a transcription reaction was performed in vitro by T7 RNA polymerase, whereupon the substrate was labeled with [α-³²P]UTP. After treatment with DNase, RNA was precipitated and dissolved in a RNase-free sterile water, the concentration was determined using a spectrophotometer, and NS5B + strand RNA was obtained.

0.05 pmole of the NS5B + strand RNA and 50 pmole of the ribozyme were mixed, and the mixture was reacted in a solution containing 50 mM Tris-HCl (pH 7.5) and 20 mM MgCl₂ at 37°C for two hours. The reaction was stopped by adding EDTA to a final concentration of 50 mM. An equal amount of an electrophoresis buffer containing 50 mM EDTA at a final concentration and 95% formamide was added. This was subjected to denatured polyacrylamide gel electrophoresis and then analyzed by autoradiography. The result, as shown in Figure 17, demonstrated that when the loop structure becomes larger in length, cleaving activity can be attenuated as expected.

### Example 11

### Setting up an evaluation system for ribozyme cleaving activity in the cell

For the purpose of detecting RNA cleaved by a ribozyme in the cell, a system was set up in which an in vitro-cleaved RNA fragment is mixed with cell-derived RNA and then detected.

After pBKCMVNS5B(+) was digested with a restriction enzyme EcoRI, and purified after inactivation of RNase by Proteinase K. Then, using an in vitro transcription kit (Ambion), a transcription reaction was performed in vitro by T7 RNA polymerase. After treatment with DNase, RNA was precipitated and dissolved in a RNase-free sterile water, the concentration was determined using a spectrophotometer, and NS5B + strand RNA was obtained.

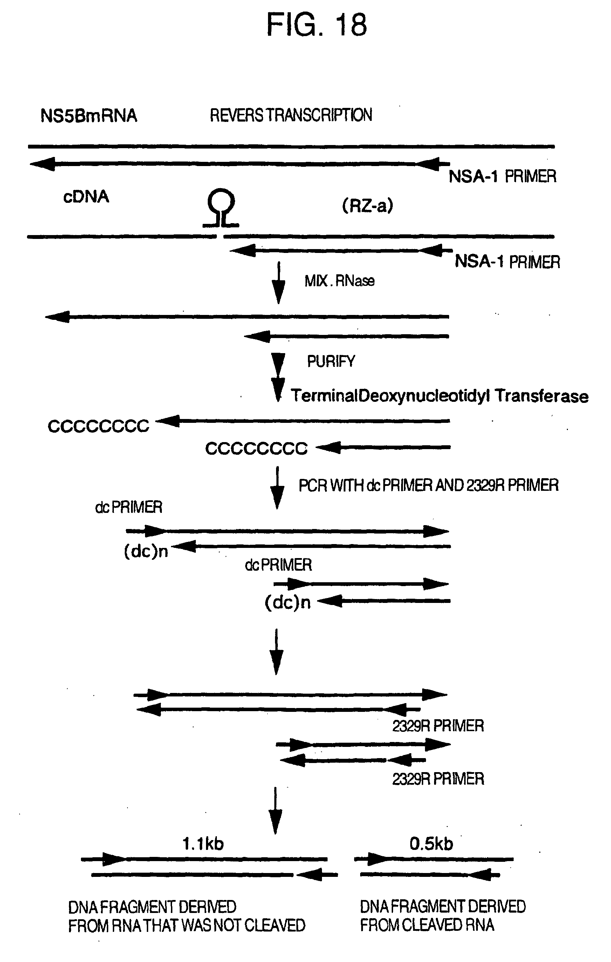

In order to detect RNA cleaved by ribozymes, the procedure shown in Figure 18 was followed.

0.15 pmole of the NS5B + strand RNA and 150 pmole of the chemically synthesized ribozyme Rz-a were mixed, and the mixture was reacted in a solution containing 50 mM Tris-HCl (pH 7.5) and 20 mM MgCl₂ at 37°C for two hours. After the reaction mixture was heated to 95°C, it was cooled, and mixed with 1 µg of total RNA of COS1 cells expressing NS5B + strand RNA. Then, using 5' RACE System for Rapid Amplification of cDNA Ends, Version 2.0 (a kit manufactured by Life Technology) and the NSA-1 primer (5'-GATAGTACACCCGTTTGCCTGATG-3', SEQ ID NO: 84), reverse transcription was performed. After digesting RNA with RNase, it was purified, and subjected to a dC addition reaction using Terminal deoxynucleotidyl Transferase (TdT). Using the dC primer (Abridged Anchor Primer in the kit) and the 2329R primer (5'-GCCTCGGGGTCCAAGTCACAA-3', SEQ ID NO: 85), PCR was performed. 'The 1.1 kb DNA fragment derived from RNA that was not cleaved by the ribozyme and a 0.5 kb DNA fragment that was cleaved were separated on electrophoresis.

As a result, a fragment possibly cleaved by ribozymes and a fragment possibly not cleaved were detected (Figure 19).

In order to evaluate the cleaving activity of a prodrug ribozyme in the cell, the following experiment may be conducted.

A plasmid integrating a SV40-derived intervening sequence in the ribozyme sequence is introduced into COS1 cells expressing HCV NS5B + strand RNA using FuGENE6 (Boehringer Mannheim). On the day after 8 × 10⁵ cells are plated on a 10 cm petri dish, 6 µg of DNA is introduced into the cells. Forty eight hours later RNA is collected by ISOGEN (Nippon Gene), and DNA is digested with DNase. Using the collected RNA, in the above procedures, a band possibly derived from RNA cleaved by ribozyme and a band possibly derived from RNA not cleaved are detected, and thereby the cleaving activity of prodrug ribozymes in an intervening sequence-inserted form in the cell can be confirmed.

### Example 12

### Investigation on splicing in the cell.

In order to confirm that a prodrug ribozyme i.e. a ribozyme in which an intervening sequence was inserted actually undergoes splicing in the cell, plasmid vectors expressing a prodrug ribozyme and a mature ribozyme after splicing out were constructed.

First, vectors pcΔNRz-aS3 and pcΔNRz-aS4 that express prodrug ribozyme Rz-aS3 or Rz-aS4 were constructed, respectively.

Rz-aS3 (SEQ ID NO: 86)
5'-AUG UCG UAC UGA AGA GGC AAG GUA AAU AUA AAA UUU UUA
AGU GUA UAA UGU GUU AAA CUA CUG AUU CUA AUU GUU UGU GUA
UUU UAG AUG CCG AAA GGG CCA U-3'

Rz-aS4 (SEQ ID NO: 87)
5'-AUG UCG UAC UGA UGA GGA AUA AGG UAA AUA UAA AAU UUU
UAA GUG UAU AAU GUG UUA AAC UAC UGA UUC UAA UUG UUU GUG
UAU UUU AGA UUC CGA AAG GGC CAU-3'

In this case, PCR was performed, using two oligonucleotides encoding each ribozyme sequence having a restriction enzyme recognition sequence at its end and an SV40-derived intervening sequence as primers against SV40 DNA (Gibco) as a template. The upstream primer used in the PCR for Rz-aS3 construction was SEQ ID NO: 88, and the downstream primer was SEQ ID NO: 89. The upstream primer used in the PCR for Rz-aS4 construction was SEQ ID NO: 90, and the downstream primer was SEQ ID NO: 91. Then, after cleaving with restriction enzymes NheI and HindIII, fragments were purified, and inserted into restriction enzyme NheI and HindIII sites of pcDNA3.1(+) that has a mutation at the NotI cleavage site.

Subsequently, vectors pcΔNRz-aS(-)3 and pcΔNRz-aS(-) 4 that express mature ribozyme Rz-a(-)S3 or Rz-a(-)S4 were constructed.

In this case, two oligonucleotides encoding a ribozyme molecule were chemically synthesized. The two oligonucleotides for Rz-a(-)S3 construction are represented by SEQ ID NO: 92 and 93, and the two oligonucleotides for Rz-a(-)S4 construction are represented by SEQ ID NO: 94 and 95. Then, oligonucleotides were purified, the 5'-ends were phosphorylated, and then annaled. They were then inserted into restriction enzyme NheI and HindIII sites of pcDNA3.1(+) that has a mutation at the NotI cleavage site.

The above plasmid was introduced into COS1 cells expressing HCV NS5B + strand RNA using FuGENE6 (Boehringer Mannheim). On the day after 8 × 10⁵ cells are plated on a 10 cm petri dish, 6 µg of DNA was introduced into the cells. Forty eight hours later total RNA was collected by ISOGEN (Nippon Gene).

After digesting DNA with DNase, reverse transcription and PCR were performed using 'the GeneAmp RNA PCR Core Kit (Perkin-Elmer). As a primer in the reverse transcription reaction, a random 9mer primer (Takara Shuzo) was used. As a primer for PCR, pcDNA3.1BGH/reverse primer (SEQ ID NO: 55) and primers that specifically recognize the intervening sequence (RzS3/S-Int. primer (SEQ ID NO: 96), RzS4/S-Int. primer (SEQ ID NO: 97)) or primers that specifically recognize the spliced-out mature ribozyme (RzS3/S-Exon. primer (SEQ ID NO: 98), RzS4/S-Exon. primer (SEQ ID NO: 99)) were used. By subjecting the PCR products to electrophoresis, fragments were confirmed.

As a result, when an intervening sequence-specific primer was used, fragments were confirmed only in the lanes of RNA derived from the cell which an intervening sequence-inserted ribozyme (prodrug ribozyme) was introduced. When mature ribozyme-specific primer was used, fragments were confirmed not only in lanes of mature ribozyme-introduced cellular RNA but also in lanes of RNA derived from the cell which an intervening sequence-inserted ribozyme was introduced. From the above result, fragments that probably underwent splicing were detected (Figure 20).

### Example 13

### Investigation of the cleaving activity of ribozymes in which an insertion sequence was inserted into the stem and the active region

Ribozymes in the form that permits the insertion of an intervening sequence into the stem region site of ① and ② in Figure 10 and ribozymes in which an intervening sequence such as SV40 small T Intron has been inserted into ① and ② in Figure 10 are synthesized. Furthermore, ribozymes having a profile that permits the insertion of an intervening sequence into the loop region site required for cleaving activity of ⑦ in Figure 10 and ribozymes in which an intervening sequence such as SV40 small +T Intron has been inserted into ⑦ in Figure 10 are synthesized. The evaluation of the cleaving activity reveals that no cleaving activity is observed probably because an intervening sequence is inserted into the stem site or in the proximity of the active site of the ribozyme, it is believed, it is difficult for the ribozyme to take a structure having a cleaving activity.

For the detection, a method similar to that of Example 10 is used. Thus, a ribozyme-expressing vector is constructed, and using an in vitro transcription a ribozyme molecule and HCV NS5B + strand RNA that could be a substrate are synthesized. After mixing NS5B + strand RNA and the ribozyme, and allowing to react at 37°C for two hours in a solution containing 50 mM Tris-HCl (pH 7.5) and 20 mM MgCl₂, EDTA at a final concentration of 50 mM is added to stop the reaction. An equal amount of electrophoresis buffer containing EDTA at a final concentration of 50 mM and 95% formamide is added. This is subjected to denatured polyacrylamide gel electrophoresis and then analyzed autoradiography.

As a result, it is confirmed that the ribozyme in which an intervening sequence is inserted into the stem site or in the proximity of the active site has no cleaving activity, and only the ribozyme having no intervening sequence has the cleaving activity.

### INDUSTRIAL APPLICABILITY

In accordance with the present invention, there is provided a gene encoding a pharmaceutically useful prodrug ribozyme, an expression vector having said gene, a prodrug ribozyme, and a pharmaceutical composition containing said gene as an active ingredient.

### Sequence listing free text

SEQ ID NO: 1 is the base sequence of cDNA to HCV NSSB + strand RNA that is a substrate for a ribozyme, and an amino acid sequence encoded thereby.

SEQ ID NO: 2-18 are the target sequences, in the substrate HCV NS5B + strand RNA, estimated to be cleaved by a ribozyme.

SEQ ID NO: 19-35 are the base sequences of ribozyme molecule RNAs to the target sequences of SEQ ID NO: 2-18.

SEQ ID NO: 36-41 are the base sequences of mature ribozyme molecule RNAs in which an intervening sequence has been eliminated.

SEQ ID NO: 42-50 are the base sequences of the target sequences of cleavable ribozymes.

SEQ ID NO: 51 is the base sequence of RNA that contributes to stabilization of histone mRNA commonly present at the 3'-end of histone mRNA.

SEQ ID NO: 52 and 53 are the base sequences of oligonucleotides used for creating mature ribozyme molecules in which an intervening sequence has been eliminated.

SEQ ID NO: 54 and 55 are the base sequence of T7 promoter primer and pcDNA3.1(+)/BGH reverse primer, respectively.

SEQ ID NO: 56 and 57 are the base sequences of oligonucleotides corresponding to SV40 small T intron region and a spacer region.

SEQ ID NO: 58-63 are the target sequences, in the substrate HCV NS5B RNA, estimated to be cleaved by a ribozyme.

SEQ ID NO: 64-69 are the base sequences of ribozyme molecule RNAs corresponding to the target sequences of SEQ ID NO: 58-63.

SEQ ID NO: 70-74 are the base sequences of the target sequences that can be cleaved by ribozymes.

SEQ ID NO: 75 is the base sequence of ribozyme molecule RNA that was altered based on the sequence of the adjacent region of SV40 small T intervening sequence.

SEQ ID NO: 76 is the base sequence of ribozyme molecule RNA that has a sequence homologous to the sequence of the adjacent region of SV40 small T intervening sequence.

SEQ ID NO: 77 is the base sequence of ribozyme molecule RNA into which an intervening sequence can be inserted.

SEQ ID NO: 78 is the base sequence of ribozyme molecule RNA into which an intervening sequence has been inserted.

SEQ ID NO: 79 is the base sequence of ribozyme molecule RNA into which a spacer sequence has been inserted.

SEQ ID NO: 80 and 81 are the base sequences of oligonucleotides used for creating the ribozyme molecule of SEQ ID NO: 78.

SEQ ID NO: 82 and 83 are the base sequences of oligonucleotides used for creating the ribozyme molecule of SEQ ID NO: 79.

SEQ ID NO: 84 and 85 are the base sequences of NSA-1 primer and 2329R primer, respectively.

SEQ ID NO: 86 and 87 are the base sequences of prodrug ribozyme molecule RNAs.

SEQ ID NO: 88-91 are the base sequences of primers used for creating prodrug ribozyme molecules.

SEQ ID NO: 92-95 are the base sequences of oligonucleotides used for creating ribozyme molecules.

SEQ ID NO: 96 and 97 are the base sequences of primers that specifically recognize an intervening sequence.

SEQ ID NO: 98 and 99 are the base sequences of primers that specifically recognize a mature ribozyme.

## Claims

1. A gene encoding a prodrug ribozyme having one or a plurality of the following features:
(a) containing, in the ribozyme sequence, an intervening sequence that is eliminated by splicing, and
(b) having no RNA-cleaving activity.

2. The gene according to claim 1 that is a small ribozyme selected from a hammerhead ribozyme, a hairpin ribozyme or a delta ribozyme, and a large ribozyme selected from Group I ribozyme, Group II ribozyme and RNaseP, or a derivative thereof.

3. The gene according to claim 2 in which the ribozyme is a small ribozyme selected from a hammerhead ribozyme, a hairpin ribozyme, and a delta ribozyme, or a derivative thereof.

4. The gene according to claim 2 in which the intervening sequence is present at the loop site or the stem site of the ribozyme sequence.

5. The gene according to claim 1 in which the intervening sequence is an intervening sequence derived from a virus or an intervening sequence derived from an eukaryote.

6. The gene according to claim 5 in which the intervening sequence is an intervening sequence derived from a virus selected from papovavirus, adenovirus, herpesvirus, papilloma virus, retrovirus, or lentivirus.

7. The gene according to claim 5 in which the intervening sequence is an intervening sequence derived from SV40, an intervening sequence derived from the rabbit β-globin gene, or an intervening sequence derived from the mouse albumin gene.

8. The gene according to claim 1 in which the intervening sequence contained in the ribozyme sequence is flanked by a spacer sequence on both ends, wherein said spacer sequence comprises an exon partial sequence flanking the 5'-end and the 3'-end of said intervening sequence in a eukaryote or a virus from which said intervening sequence is derived.

9. The gene according to claim 1 in which a proximate region on both ends of the intervening sequence contained in the ribozyme sequence has the following sequence:
(a) 5'-end terminal sequence: 5'-(A/C)AGGU(A/G)AGU
(b) 3'-end terminal sequence: PyPyPyPyPyPyPyPyPyPyPyN(C/U)AG(G/A)-3' (Py represents U or C, and N represents any residue).

10. The gene according to claim 1 in which the ribozyme is a ribozyme having an activity of cleaving virus-derived RNA.

11. The gene according to claim 10 in which the ribozyme is a ribozyme having an activity of cleaving HCV RNA.

12. The gene according to claim 11 in which the ribozyme is a ribozyme that recognizes HCV RNA containing any of the sequences as set forth in SEQ ID NO: 42-50 or a sequence in which several bases thereof have been replaced.

13. The gene according to claim 1 encoding multiple prodrug ribozymes.

14. The gene according to claim 13 in which the intervening sequence contained in each prodrug ribozyme is different from one another.

15. An expression vector having the gene according to any of claims 1 to 14.

16. The expression vector according to claim 15 having a tissue specific promoter.

17. The expression vector according to claim 15 which contains a sequence having an activity of activating the extranuclear transport of mRNA or an activity of stabilizing mature mRNA.

18. One or multiple prodrug ribozyme having one or a plurality of the following features:
(a) containing, in the ribozyme sequence, an intervening sequence that is eliminated by splicing, and
(b) having no RNA-cleaving activity.

19. A pharmaceutical composition comprising the gene according to claim 1 as an active ingredient.

20. A therapeutic agent for treatment of viral diseases comprising the gene according to claim 10 as an active ingredient.

21. A therapeutic agent for treatment of HCV diseases comprising the gene according to claim 11 as an active ingredient.

22. A method of producing, in the cytoplasm, a mature ribozyme having an RNA-cleaving activity, said method comprising introducing the gene according to claim 1 into an eukaryotic cell.


SEQUENCE LISTING

<110> Sumitomo Pharmaceuticals Company, Limited

<120> Prodrug Ribozyme

<130> E4778-00

<150> JP 10-249900
<151> 1998-9-3

<160> 99

<210> 1
<211> 1779
<212> DNA
<213> HCV

<220>
<221> CDS
<222> (1)...(1779)

<400> 1
   atg tcg atg tcc tac aca tgg aca ggc gcc tta att aca cca tgc gcc 48
   Met Ser Met Ser Tyr Thr Trp Thr Gly Ala Leu Ile Thr Pro Cys Ala
   5 10 15
   gcg gag gag agc aag ctg ccc att aat gcg ctg agc aac cct ttg ctg 96
   Ala Glu Glu Ser Lys Leu Pro Ile Asn Ala Leu Ser Asn Pro Leu Leu
   20 25 30
   cgc cac cac aac atg gtc tat gcc aca aca tcc cgc agc gca agc cag 144
   Arg His His Asn Met Val Tyr Ala Thr Thr Ser Arg Ser Ala Ser Gln
   35 40 45
   cgg cag aaa aag gtc aca ttt gac aga ctg caa gtc ctg gat gac cac 192
   Arg Gln Lys Lys Val Thr Phe Asp Arg Leu Gln Val Leu Asp Asp His
   50 55 60
   tac cgg gac gtg ctg aag ggg atg aag gcg aag gcg tcc aca gtt aag 240
   Tyr Arg Asp Val Leu Lys Gly Met Lys Ala Lys Ala Ser Thr Val Lys
   65 70 75 80
   gct aaa ctt cta tct gta gag gaa gcc tgc aag ctg acg ccc cca cac 288
   Ala Lys Leu Leu Ser Val Glu Glu Ala Cys Lys Leu Thr Pro Pro His
   85 90 95
   tcg gcc aga tct aaa ttt ggc tac ggg gca aag gac gtc cgg agc ctg 336
   Ser Ala Arg Ser Lys Phe Gly Tyr Gly Ala Lys Asp Val Arg Ser Leu
   100 105 110
   tcc agc aag gcc gtt aac cac atc cgc tcc gtg tgg aag gac ttg ctg 384
   Ser Ser Lys Ala Val Asn His Ile Arg Ser Val Trp Lys Asp Leu Leu
   115 120 125
   gaa gac act gag aca cca att gac acc acc atc atg gca aaa aat gag 432
   Glu Asp Thr Glu Thr Pro Ile Asp Thr Thr Ile Met Ala Lys Asn Glu
   130 135 140
   gtt ttc tgt gtt caa cca gag aaa gga ggc cgc aag cca gct cgc ctt 480
   Val Phe Cys Val Gln Pro Glu Lys Gly Gly Arg Lys Pro Ala Arg Leu
   145 150 155 160
   atc gta ttc cca gac ttg ggg gtt cgt gtg tgc gag aaa atg gcc ctc 528
   Ile Val Phe Pro Asp Leu Gly Val Arg Val Cys Glu Lys Met Ala Leu
   165 170 175
   tac gac gtg gtc tcc act ctt cct cag gcc gtg atg ggc tcc tca tac 576
   Tyr Asp Val Val Ser Thr Leu Pro Gln Ala Val Met Gly Ser Ser Tyr
   180 185 190
   gga ttc cag tac tcc cct gga cag cgg gtc gag ttc ctg gtg aat gcc 624
   Gly Phe Gln Tyr Ser Pro Gly Gln Arg Val Glu Phe Leu Val Asn Ala
   195 200 205
   tgg aag tca aag aag agc cct atg ggc ttt gca tat gac acc cgc tgt 672
   Trp Lys Ser Lys Lys Ser Pro Met Gly Phe Ala Tyr Asp Thr Arg Cys
   210 215 220
   ttt gac tca acg gtc acc gag aac gac atc cgt act gag gag tca att 720
   Phe Asp Ser Thr Val Thr Glu Asn Asp Ile Arg Thr Glu Glu Ser Ile
   225 230 235 240
   tat caa tgt tgt gac ttg gac ccc gag gcc aga cag gcc ata agg tcg 768
   Tyr Gln Cys Cys Asp Leu Asp Pro Glu Ala Arg Gln Ala Ile Arg Ser
   245 250 255
   ctc aca gag cgg ctt tat atc ggg ggc ccc ctg act aat tca aag ggg 816
   Leu Thr Glu Arg Leu Tyr Ile Gly Gly Pro Leu Thr Asn Ser Lys Gly
   260 265 270
   cag aac tgc ggt tat cgc cgg tgc cgc gtc agc ggc gtg ctg acg act 864
   Gln Asn Cys Gly Tyr Arg Arg Cys Arg Val Ser Gly Val Leu Thr Thr
   275 280 285
   agc tgc ggt aat acc ctc aca tgt tac ttg aag gcc tct gca gcc tgt 912
   Ser Cys Gly Asn Thr Leu Thr Cys Tyr Leu Lys Ala Ser Ala Ala Cys
   290 295 300
   cga gct gca aag ctc cag gac tgc acg atg ctt gtg tgc gga gac gac 960
   Arg Ala Ala Lys Leu Gln Asp Cys Thr Met Leu Val Cys Gly Asp Asp
   305 310 315 320
   ctt gtc gtt atc tgt gat agc gcg gga act cag gag gac gcg gcg agc 1008
   Leu Val Val Ile Cys Asp Ser Ala Gly Thr Gln Glu Asp Ala Ala Ser
   325 330 335
   cta cga gtc ttc acg gag gct atg act agg tac tct gcc ccc ccc ggg 1056
   Leu Arg Val Phe Thr Glu Ala Met Thr Arg Tyr Ser Ala Pro Pro Gly
   340 345 350
   gac ccg ccc caa cca gaa tac gac ttg gag ctg ata aca tca tgc tcc 1104
   Asp Pro Pro Gln Pro Glu Tyr Asp Leu Glu Leu Ile Thr Ser Cys Ser
   355 360 365
   tcc aat gtg tcg gtc gcg cac gac gca tca ggc aaa cgg gtg tac tat 1152
   Ser Asn Val Ser Val Ala His Asp Ala Ser Gly Lys Arg Val Tyr Tyr
   370 375 380
   ctc acc cgt gac ccc acc acc ccc cta gcg cgg gct gcg tgg gag aca 1200
   Leu Thr Arg Asp Pro Thr Thr Pro Leu Ala Arg Ala Ala Trp Glu Thr
   385 390 395 400
   gct aga cac act cca gtc aac tcc tgg cta ggc aac atc atc atg tac 1248
   Ala Arg His Thr Pro Val Asn Ser Trp Leu Gly Asn Ile Ile Met Tyr
   405 410 415
   gcg ccc acc tta tgg gca agg atg att ctg atg acc cac ttc ttc tcc 1296
   Ala Pro Thr Leu Trp Ala Arg Met Ile Leu Met Thr His Phe Phe Ser
   420 425 430
   atc ctt cta gcc cag gag caa ctt gaa aaa gcc cta gat tgt cag atc 1344
   Ile Leu Leu Ala Gln Glu Gln Leu Glu Lys Ala Leu Asp Cys Gln Ile
   435 440 445
   tac ggg gcc act tac tcc att gag cca ctt gac cta cct cag atc att 1392
   Tyr Gly Ala Thr Tyr Ser Ile Glu Pro Leu Asp Leu Pro Gln Ile Ile
   450 455 460
   caa cga ctc cac ggt ctt agc gca ttt tca ctc cat agt tac tct cca 1440
   Gln Arg Leu His Gly Leu Ser Ala Phe Ser Leu His Ser Tyr Ser Pro
   465 470 475 480
   ggt gag atc aat agg gtg gct tca tgc ctc agg aaa ctt ggg gta ccg 1488
   Gly Glu Ile Asn Arg Val Ala Ser Cys Leu Arg Lys Leu Gly Val Pro
   485 490 495
   ccc ttg cga gtc tgg aga cat cgg gcc aga agc gtc cgc gct aag cta 1536
   Pro Leu Arg Val Trp Arg His Arg Ala Arg Ser Val Arg Ala Lys Leu
   500 505 510
   ctg tcc cag ggg ggg agg gcc gcc acc tgt ggc aaa tac ctc ttc aac 1584
   Leu Ser Gln Gly Gly Arg Ala Ala Thr Cys Gly Lys Tyr Leu Phe Asn
   515 520 525
   tgg gca gta aag acc aag ctc aaa ctc act cca atc cca gaa gcg tcc 1632
   Trp Ala Val Lys Thr Lys Leu Lys Leu Thr Pro Ile Pro Glu Ala Ser
   530 535 540
   cag ctg gac ttg tcc ggc tgg ttc gtt gct ggt tac agc ggg gga gac 1680
   Gln Leu Asp Leu Ser Gly Trp Phe Val Ala Gly Tyr Ser Gly Gly Asp
   545 550 555 560
   ata tat cac agc ctg tct cgt gcc cga ccc cgc tgg ttc atg tgg tgc 1728
   Ile Tyr His Ser Leu Ser Arg Ala Arg Pro Arg Trp Phe Met Trp Cys
   565 570 575
   cta ctc cta ctt tcc gta ggg gta ggc atc tac ctg ctc ccc aac cga 1776
   Leu Leu Leu Leu Ser Val Gly Val Gly Ile Tyr Leu Leu Pro Asn Arg
   580 585 590
   tga 1779

<210> 2
<211> 17
<212> RNA
<213> HCV

<400> 2
auggcccucu acgacgu 17

<210> 3
<211> 17
<212> RNA
<213> HCV

<400> 3
uacggauucc aguacuc 17

<210> 4
<211> 17
<212> RNA
<213> HCV

<400> 4
augcuccucc aaugugu 17

<210> 5
<211> 17
<212> RNA
<213> HCV

<400> 5
uaccucuuca acugggc 17

<210> 6
<211> 17
<212> RNA
<213> HCV

<400> 6
guagggguag gcaucua                                                    17

<210> 7
<211> 17
<212> RNA
<213> HCV

<400> 7
ucaacgguca ccgagaa                                                    17

<210> 8
<211> 17
<212> RNA
<213> HCV

<400> 8
cggaggcuau gacuagg                                                    17

<210> 9
<211> 17
<212> RNA
<213> HCV

<400> 9
gagcugauaa caucaug                                                    17

<210> 10

<211> 17

<212> RNA

<213> HCV

<400> 10

aacaucauca uguacgc 17

<210> 11

<211> 17

<212> RNA

<213> HCV

<400> 11

accaagcuca aacucac 17

<210> 12

<211> 17

<212> RNA

<213> HCV

<400> 12

ggucacauuu gacagac 17

<210> 13

<211> 17

<212> RNA

<213> HCV

<400> 13

ggcccucuac gacgugg 17

<210> 14

<211> 17

<212> RNA

<213> HCV

<400> 14

gucguuaucu gugauag 17

<210> 15

<211> 17

<212> RNA

<213> HCV

<400> 15

accagaauac gacuugg 17

<210> 16

<211> 17

<212> RNA

<213> HCV

<400> 16

aggaaacuug ggguacc 17

<210> 17

<211> 17

<212> RNA

<213> HCV

<400> 17
aacucacucc aaucccg                                                    17

<210> 18
<211> 17
<212> RNA
<213> HCV

<400> 18
cagccugucu cgugccc                                                    17

<210> 19
<211> 38
<212> RNA
<213> Artificial Sequence

<220>
<223> Designed Ribozyme Molecule

<400> 19
augucguacu gaugaguccg ugaggacgaa agggccau                             38

<210> 20
<211> 38
<212> RNA
<213> Artificial Sequence

<220>

<223> Designed Ribozyme Molecule

<400> 20

gaguauugcu gaugaguccg ugaggacgaa aauccgua 38

<210> 21

<211> 38

<212> RNA

<213> Artificial Sequence

<220>

<223> Designed Ribozyme Molecule

<400> 21

auacguugcu gaugaguccg ugaggacgaa aggagcau 38

<210> 22

<211> 38

<212> RNA

<213> Artificial Sequence

<220>

<223> Designed Ribozyme Molecule

<400> 22

gcccaguucu gaugaguccg ugaggacgaa aagaggua 38

<210> 23

<211> 38

<212> RNA

<213> Artificial Sequence

<220>

<223> Designed Ribozyme Molecule

<400> 23

uagaugcccu gaugaguccg ugaggacgaa acuccuac 38

<210> 24

<211> 38

<212> RNA

<213> Artificial Sequence

<220>

<223> Designed Ribozyme Molecule

<400> 24

uucucggucu gaugaguccg ugaggacgaa acuguuga 38

<210> 25

<211> 38

<212> RNA

<213> Artificial Sequence

<220>

<223> Designed Ribozyme Molecule

<400> 25

uguggucacu gaugaguccg ugaggacgaa agccuccg                                38

<210> 26

<211> 38

<212> RNA

<213> Artificial Sequence

<220>

<223> Designed Ribozyme Molecule

<400> 26

caugaugucu gaugaguccg ugaggacgaa aucagcuc                                38

<210> 27

<211> 38

<212> RNA

<213> Artificial Sequence

<220>

<223> Designed Ribozyme Molecule

<400> 27

gcguacaucu gaugaguccg ugaggacgaa augauguu                                38

<210> 28

<211> 38

<212> RNA

<213> Artificial Sequence

<220>

<223> Designed Ribozyme Molecule

<400> 28

gugaguuucu gaugaguccg ugaggacgaa aguuuggu 38

<210> 29

<211> 38

<212> RNA

<213> Artificial Sequence

<220>

<223> Designed Ribozyme Molecule

<400> 29

gucugucacu gaugaguccg ugaggacgaa aggugacc 38

<210> 30

<211> 38

<212> RNA

<213> Artificial Sequence

<220>

<223> Designed Ribozyme Molecule

<400> 30

ucaugucgcu gaugaguccg ugaggacgaa agagggcc 38

<210> 31
<211> 38
<212> RNA
<213> Artificial Sequence

<220>
<223> Designed Ribozyme Molecule

<400> 31
cuuucacacu gaugaguccg ugaggacgaa augacgac 38

<210> 32
<211> 38
<212> RNA
<213> Artificial Sequence

<220>
<223> Designed Ribozyme Molecule

<400> 32
ccaggucgcu gaugaguccg ugaggacgaa auucuggu 38

<210> 33
<211> 38
<212> RNA
<213> Artificial Sequence

<220>
<223> Designed Ribozyme Molecule

<400> 33
ggugcccccu gaugaguccg ugaggacgaa aguuuccu                                38

<210> 34
<211> 38
<212> RNA
<213> Artificial Sequence

<220>
<223> Designed Ribozyme Molecule

<400> 34
ugggauugcu gaugaguccg ugaggacgaa agugaguu                                38

<210> 35
<211> 38
<212> RNA
<213> Artificial Sequence

<220>
<223> Designed Ribozyme Molecule

<400> 35
gggcaugacu gaugaguccg ugaggacgaa acaggcug                                38

<210> 36
<211> 64
<212> RNA

<213> Artificial Sequence

<220>

<223> Basic Sequence of Ribozyme Molecule

<400> 36

augucguacu gaugaguccg ugcggccgcg gaauccuuag cggccgcgag gacgaaaggg 60

ccau 64

<210> 37

<211> 64

<212> RNA

<213> Artificial Sequence

<220>

<223> Basic Sequence of Ribozyme Molecule

<400> 37

gaguauugcu gaugaguccg ugcggccgcg gaauccuuag cggccgcgag gacgaaaauc 60

cgua 64

<210> 38

<211> 64

<212> RNA

<213> Artificial Sequence

<220>

<223> Basic Sequence of Ribozyme Molecule

<400> 38

uucucggucu gaugaguccg ugcggccgcg gaauccuuag cggccgcgag gacgaaacug 60

uuga 64

<210> 39

<211> 64

<212> RNA

<213> Artificial Sequence

<220>

<223> Basic Sequence of Ribozyme Molecule

<400> 39

gcguacaucu gaugaguccg ugcggccgcg gaauccuuag cggccgcgag gacgaaauga 60

uguu 64

<210> 40

<211> 64

<212> RNA

<213> Artificial Sequence

<220>

<223> Basic Sequence of Ribozyme Molecule

<400> 40

ccaggucgcu gaugaguccg ugcggccgcg gaauccuuag cggccgcgag gacgaaauuc 60

uggu 64

<210> 41

<211> 64

<212> RNA

<213> Artificial Sequence

<220>

<223> Basic Sequence of Ribozyme Molecule

<400> 41

ugggauugcu gaugaguccg ugcggccgcg gaauccuuag cggccgcgag gacgaaagug 60

aguu 64

<210> 42

<211> 17

<212> RNA

<213> Artificial Sequence

<220>

<223> Target Sequence of Ribozyme Molecule

<400> 42

auggcycuyu aygayru 17

<210> 43

<211> 17

<212> RNA

<213> Artificial Sequence

<220>

<223> Target Sequence of Ribozyme Molecule

<400> 43
uayggruucc aruacuc 17

<210> 44
<211> 17
<212> RNA
<213> Artificial Sequence

<220>
<223> Target Sequence of Ribozyme Molecule

<400> 44
ucaacvguca cbgagar 17

<210> 45
<211> 17
<212> RNA
<213> Artificial Sequence

<220>
<223> Target Sequence of Ribozyme Molecule

<400> 45
aayauyauym wguwygc 17

<210> 46
<211> 17
<212> RNA

<213> Artificial Sequence

<220>
<223> Target Sequence of Ribozyme Molecule

<400> 46
ggcycuyuay gayrusr                                                    17

<210> 47
<211> 17
<212> RNA
<213> Artificial Sequence

<220>
<223> Target Sequence of Ribozyme Molecule

<400> 47
guyguyaucu swgarag                                                    17

<210> 48
<211> 17
<212> RNA
<213> Artificial Sequence

<220>
<223> Target Sequence of Ribozyme Molecule

<400> 48
accrgaauay gacyugg                                                    17

<210> 49
<211> 17
<212> RNA
<213> Artificial Sequence

<220>
<223> Target Sequence of Ribozyme Molecule

<400> 49
agraaacuug grgyvcc                                                    17

<210> 50
<211> 17
<212> RNA
<213> Artificial Sequence

<220>
<223> Target Sequence of Ribozyme Molecule

<400> 50
aacucacucc awubccv                                                    17

<210> 51
<211> 16
<212> RNA
<213> Artificial Sequence

<220>

<223> Histone mRNA Stabilizing Sequence

<400> 51
ggyyyuuyun arrrcc                                                    16

<210> 52
<211> 70
<212> DNA
<213> Artificial Sequence

<220>
<223> Oligonucleotide Encoding Matured Ribozyme

<400> 52
agcttatgtc gtactgatga gtccgtgcgg ccgcggaatc cttagcggcc gcgaggacga    60
aagggccata                                                           70

<210> 53
<211> 70
<212> DNA
<213> Artificial Sequence

<220>
<223> Oligonucleotide Encoding Matured Ribozyme

<400> 53
agcttatggc cctttcgtcc tcgcggccgc taaggattcc gcggccgcac ggactcatca    60
gtacgacata                                                           70

<210> 54

<211> 20

<212> DNA

<213> Artificial Sequence

<220>

<223> T7 Promoter Primer

<400> 54

taatacgact cactataggg 20

<210> 55

<211> 20

<212> DNA

<213> Artificial Sequence

<220>

<223> pcDNA3. 1/BGH Riverse Primer

<400> 55

tagaaggcac agtcgagg 18

<210> 56

<211> 83

<212> DNA

<213> Artificial Sequence

<220>

<223> Oligonucleotide Encoding SV40 Small T Intron

<400> 56

ggccgcggaa tctaaaatac acaaacaatt agaatcagta gtttaacaca ttatacactt     60

aaaaatttta tatttacctt agc                                            83

<210> 57

<211> 83

<212> DNA

<213> Artificial Sequence

<220>

<223> Oligonucleotide Encoding SV40 Small T Intron

<400> 57

ggccgctaag gtaaatataa aatttttaag tgtataatgt gttaaactac tgattctaat     60

tgtttgtgta ttttagattc cgc                                            83

<210> 58

<211> 17

<212> RNA

<213> HCV

<400> 58

ccccaaguuu ccugagg                                                   17

<210> 59

<211> 17

<212> RNA

<213> HCV

<400> 59
cauagccucc gugaaga 17

<210> 60
<211> 17
<212> RNA
<213> HCV

<400> 60
ggaguacugg aauccgu 17

<210> 61
<211> 17
<212> RNA
<213> HCV

<400> 61
agggccauuu ucucgca 17

<210> 62
<211> 17
<212> RNA
<213> HCV

<400> 62
ugcccaguug aagaggu 17

<210> 63

<211> 17

<212> RNA

<213> HCV

<400> 63

caagucguau ucugguu 17

<210> 64

<211> 38

<212> RNA

<213> Artificial Sequence

<220>

<223> Designed Ribozyme Molecule

<400> 64

ucucaggacu gaugaguccg ugaggacgaa acuugggg 38

<210> 65

<211> 38

<212> RNA

<213> Artificial Sequence

<220>

<223> Designed Ribozyme Molecule

<400> 65

uuuucacgcu gaugaguccg ugaggacgaa aggcuaug 38

<210> 66

<211> 38

<212> RNA

<213> Artificial Sequence

<220>

<223> Designed Ribozyme Molecule

<400> 66

acggauuccu gaugaguccg ugaggacgaa aguacucu 38

<210> 67

<211> 38

<212> RNA

<213> Artificial Sequence

<220>

<223> Designed Ribozyme Molecule

<400> 67

ugcgagaacu gaugaguccg ugaggacgaa auggcucu 38

<210> 68

<211> 38

<212> RNA

<213> Artificial Sequence

<220>

<223> Designed Ribozyme Molecule

<400> 68
aucucuuucu gaugaguccg ugaggacgaa acugggcg                                38

<210> 69
<211> 38
<212> RNA
<213> Artificial Sequence

<220>
<223> Designed Ribozyme Molecule

<400> 69
aaccggaacu gaugaguccg ugaggacgaa augacuug                                38

<210> 70
<211> 17
<212> RNA
<213> Artificial Sequence

<220>
<223> Target Sequence of Ribozyme Molecule

<400> 70
cyccaaguhu ycugagr                                                       17

<210> 71
<211> 17

<212> RNA
<213> Artificial Sequence

<220>
<223> Target Sequence of Ribozyme Molecule

<400> 71
caurgcyuhc gugaarr 17

<210> 72
<211> 17
<212> RNA
<213> Artificial Sequence

<220>
<223> Target Sequence of Ribozyme Molecule

<400> 72
agrgccauyu uyucgca 17

<210> 73
<211> 17
<212> RNA
<213> Artificial Sequence

<220>
<223> Target Sequence of Ribozyme Molecule

<400> 73

ygcccaguur aagagru 17

<210> 74

<211> 17

<212> RNA

<213> Artificial Sequence

<220>

<223> Target Sequence of Ribozyme Molecule

<400> 74

cargucruhu ucygguu 17

<210> 75

<211> 38

<212> RNA

<213> Artificial Sequence

<220>

<223> Designed Ribozyme Molecule

<400> 75

augucguacu gaagaggcaa ggaugccgaa agggccau 38

<210> 76

<211> 40

<212> RNA

<213> Artificial Sequence

<220>

<223> Designed Ribozyme Molecule

<400> 76

augucguacu gaugaggaau aaggauuccg aaagggccau 40

<210> 77

<211> 38

<212> RNA

<213> Artificial Sequence

<220>

<223> Designed Ribozyme Molecule

<400> 77

augucguacu gaagagacca ggaggucgaa agggccau 38

<210> 78

<211> 113

<212> RNA

<213> Artificial Sequence

<220>

<223> Designed Ribozyme Molecule

<400> 78

augucguacu gaugaguccg uggaaucuaa aauacacaaa caauuagaau caguaguuua 60

acacauuaua cacuuaaaaa uuuuauauuu accuuagagg acgaaagggc cau 113

<210> 79

<211> 48

<212> RNA

<213> Artificial Sequence

<220>

<223> Designed Ribozyme Molecule

<400> 79

augucguacu gaugaguccg uggaauccuu agaggacgaa agggccau 48

<210> 80

<211> 68

<212> RNA

<213> Artificial Sequence

<220>

<223> Oligonucletotide for Constructing Ribozyme Molecule

<400> 80

acccaagctg gctagcatgt cgtactgatg agtccgtgga atctaaaaat acacaaacaa 60

ttagaatc 68

<210> 81

<211> 63

<212> RNA

<213> Artificial Sequence

<220>

<223> Oligonucleotide for Constructing Ribozyme Molecule

<400> 81
gctcggtacc aagcttatgg ccctttcgtc ctctaaggta aatataaaat ttttaagtgt 60
ata 63

<210> 82
<211> 54
<212> RNA
<213> Artificial Sequence

<220>
<223> Oligonucleotide for Constructing Ribozyme Molecule

<400> 82
ctagcatgtc gtactgatga gtccgtggaa tccttagagg acgaaagggc cata 54

<210> 83
<211> 54
<212> RNA
<213> Artificial Sequence

<220>
<223> Oligonucleotide for Constructing Ribozyme Molecule

<400> 83
agcttatggc cctttcgtcc tctaaggatt ccacggactc atcagtacga catg 54

<210> 84

<211> 24

<212> DNA

<213> Artificial Sequence

<220>

<223> NSA-1 Primer

<400> 84

gatagtacac ccgtttgcct gatg 24

<210> 85

<211> 21

<212> DNA

<213> Artificial Sequence

<220>

<223> 2329R Primer

<400> 85

gcctcggggt ccaagtcaca a 21

<210> 86

<211> 103

<212> RNA

<213> Artificial Sequence

<220>

<223> Prodrug Ribozyme Rz-aS3

<400> 86

augucguacu gaagaggcaa gguaaauaua aaauuuuuaa guguauaaug uguuaaacua     60

cugauucuaa uuguuugugu auuuuagaug ccgaaagggc cau                       103

<210> 87

<211> 105

<212> RNA

<213> Artificial Sequence

<220>

<223> Prodrug Ribozyme Rz-aS4

<400> 87

augucguacu gaugaggaau aagguaaaua uaaaauuuuu aaguguauaa uguguuaaac     60

uacugauucu aauuguuugu guauuuuaga uuccgaaagg gccau                     105

<210> 88

<211> 58

<212> DNA

<213> Artificial Sequence

<220>

<223> Upstream Primer for Constructing Rz-aS3

<400> 88

acccaagctg gctagcatgt cgtactgaag aggcaaggta aatataaaat ttttaagt       58

<210> 89

<211> 52

<212> DNA

<213> Artificial Sequence

<220>

<223> Downstream Primer for Constructing Rz-aS3

<400> 89

gctcggtacc aagcttatgg ccctttcggc atctaaaata cacaaacaat ta 52

<210> 90

<211> 60

<212> DNA

<213> Artificial Sequence

<220>

<223> Upstream Primer for Constructing Rz-aS4

<400> 90

acccaagctg gctagcatgt cgtactgatg aggaataagg taaatataaa atttttaagt 60

<210> 91

<211> 52

<212> DNA

<213> Artificial Sequence

<220>

<223> Downstream Primer for Constructing Rz-aS4

<400> 91

gctcggtacc aagcttatgg ccctttcgga atctaaaata cacaaacaat ta 52

<210> 92

<211> 44

<212> DNA

<213> Artificial Sequence

<220>

<223> Oligonucleotide for Constructing Rz-a(-)S3

<400> 92

ctagcatgtc gtactgaaga ggcaaggatg ccgaaagggc cata 44

<210> 93

<211> 44

<212> DNA

<213> Artificial Sequence

<220>

<223> Oligonucleotide for Constructing Rz-a(-)S3

<400> 93

agcttatggc cctttcggca tccttgcctc ttcagtacgt catg 44

<210> 94

<211> 46

<212> DNA

<213> Artificial Sequence

<220>

<223> Oligonucleotide for Constructing Rz-a(-)S4

<400> 94

ctagcatgtc gtactgatga ggaataagga ttccgaaagg gccata 46

<210> 95

<211> 46

<212> DNA

<213> Artificial Sequence

<220>

<223> Oligonucleotide for Constructing Rz-a(-)S4

<400> 95

agcttatggc cctttcggaa tccttattcc tcatcagtac gtcatg 46

<210> 96

<211> 20

<212> DNA

<213> Artificial Sequence

<220>

<223> RzS3/S-Int Primer

<400> 96

actgaagagg caaggtaaat 20

<210> 97

<211> 20

<212> DNA

<213> Artificial Sequence

<220>

<223> RzS4/S-Int Primer

<400> 97

tgatgaggaa taaggtaaat 20

<210> 98

<211> 20

<212> DNA

<213> Artificial Sequence

<220>

<223> RzS3/S-Exon Primer

<400> 98

actgaagagg caaggattcc 20

<210> 99

<211> 20

<212> DNA

<213> Artificial Sequence

<220>

<223> RzS4/S-Exon Primer

<400> 99

tgatgaggaa taaggattcc 20